# EUROPEAN PATENT APPLICATION

(11) **EP 2 192 196 A1**
(43) Date of publication of application: **02.06.2010**
(21) Application number: 08020645.1
(22) Date of filing: 27.11.2008
(51) Int. Cl.: C12Q 1/68

(54) **Prediction of lipid-metabotype-related physiological susceptibilities**

(71) Applicant: Helmholtz Zentrum München Deutsches Forschungszentrum für Gesundheit und Umwelt (GmbH), 85764 Neuherberg (DE)
(72) Inventor: The designation of the inventor has not yet been filed
(74) Representative: Vossius & Partner

(57) **Abstract**

The present invention relates to a method for determining a predisposition of a human subject for physiological susceptibilities that result from alterations in lipid metabolism, comprising determining, in a sample obtained from the human subject, the genotype of that person with respect to at least two genetic polymorphisms selected from the group consisting of a) rs2014355; wherein the minor allele is represented by a cytidine and the major allele is represented by a thymidine; b) rs11161510; wherein the minor allele is represented by a thymidine and the major allele is represented by a cytidine; c) rs2286963; wherein the minor allele is represented by a guanosine and the major allele is represented by a thymidine; d) rs174548; wherein the minor allele is represented by a guanosine and the major allele is represented by a cytidine; e) rs9393903; wherein the minor allele is represented by a adenosine and the major allele is represented by a guanosine; f) rs168622; wherein the minor allele is represented by a thymidine and the major allele is represented by a guanosine; g) rs541503; wherein the minor allele is represented by a cytidine and the major allele is represented by a thymidine; h) rs2046813; wherein the minor allele is represented by a cytidine and the major allele is represented by a thymidine; i) rs272889; wherein the minor allele is represented by a adenosine and the major allele is represented by a guanosine; and j) at least one genetic polymorphism that is in linkage disequilibrium with any of the genetic polymorphisms of (a) to (i), wherein the presence of one or two copies of the minor allele of at least two genetic polymorphisms is indicative of a predisposition for said physiological susceptibilities. Furthermore, the invention relates to a kit for determining a predisposition of a human subject for physiological susceptibilities that result from alterations in lipid metabolism.

## Description

The present invention relates to a method for determining a predisposition of a human subject for physiological susceptibilities that result from alterations in lipid metabolism, comprising determining, in a sample obtained from the human subject, the genotype of that person with respect to at least two genetic polymorphisms selected from the group consisting of a) rs2014355; wherein the minor allele is represented by a cytidine and the major allele is represented by a thymidine; b) rs11161510; wherein the minor allele is represented by a thymidine and the major allele is represented by a cytidine; c) rs2286963; wherein the minor allele is represented by a guanosine and the major allele is represented by a thymidine; d) rs174548; wherein the minor allele is represented by a guanosine and the major allele is represented by a cytidine; e) rs9393903; wherein the minor allele is represented by a adenosine and the major allele is represented by a guanosine; f) rs168622; wherein the minor allele is represented by a thymidine and the major allele is represented by a guanosine; g) rs541503; wherein the minor allele is represented by a cytidine and the major allele is represented by a thymidine; h) rs2046813; wherein the minor allele is represented by a cytidine and the major allele is represented by a thymidine; i) rs272889; wherein the minor allele is represented by a adenosine and the major allele is represented by a guanosine; and j) at least one genetic polymorphism that is in linkage disequilibrium with any of the genetic polymorphisms of (a) to (i), wherein the presence of one or two copies of the minor allele of at least two genetic polymorphisms is indicative of a predisposition for said physiological susceptibilities. Furthermore, the invention relates to a kit for determining a predisposition of a human subject for physiological susceptibilities that result from alterations in lipid metabolism.

In this specification, a number of documents including patent applications and manufacturer's manuals is cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

The metabolism of lipids, i.e. the assimilation of dietary lipids and the synthesis and degradation of lipids, plays a crucial role in cell, tissue and organ physiology as is demonstrated by a large number of genetic studies and by many human diseases that involve the disruption of lipid metabolic enzymes and pathways. Examples of such diseases include cancer, diabetes, as well as neurodegenerative and infectious diseases. However, whereas an explosion of information in the fields of genomics and proteomics has been observed, this has not been matched by a corresponding advancement of knowledge in the field of lipids, most probably because of the complexity of lipids and the lack of powerful tools for their analysis. Novel analytical approaches such as liquid chromatography and mass spectrometry for use in the systems-level analysis of lipids and their interacting partners (lipidomics) now make this field a promising area of biomedical research, with a variety of applications in drug and biomarker development.

Metabolomics is the rapidly evolving field of measuring the endogenous metabolites in a cell or body fluid (Lindon et al. 2007; Wishart et al. 2007; Assfalg et al. 2008).The metabotype or metabolic phenotype provides a readout of the metabolic state of an individual and is the product of genetic and environmental (diet, lifestyle, gut microbial activity) contributions. Analyzing metabolites (small molecules <1 kDa) in body fluids such as urine and plasma using various spectroscopic methods provides knowledge of the metabotype. Such metabolic profiles provide information reflecting true functional end-points of biological events while other functional genomics technologies such as transcriptomics and proteomics, though highly valuable, merely indicate the potential cause for phenotypic response. Therefore they cannot necessarily predict drug effects, toxicological response or disease states at the phenotype level unless functional validation is added. Metabolomics bridges this information gap by depicting in particular such functional information since metabolite differences in biological fluids and tissues provide the closest link to the various phenotypic responses. In general, phenotype is not necessarily predicted by genotype. The gap between genotype and phenotype is spanned by many biochemical reactions, each with individual dependencies to various influences, including drugs, nutrition and environmental factors. In this chain of biomolecules from the genes to phenotype, metabolites are the quantifiable molecules with the closest link to phenotype. Many phenotypic and genotypic states, such as a toxic response to a drug or disease prevalence are predicted by differences in the concentrations of functionally relevant metabolites within biological fluids and tissue.

Genome-wide association (GWA) studies have recently been used to identifiy a number of genetic polymorphisms that convey an increased risk for developing diabetes, coronary artery disease, rheumatoid arthritis, and other common diseases (Samani et al. 2007; Zeggini et al. 2008). However, the effect size of genetic associations with clinical phenotypes is often small. Therefore, large populations need to be screened in order to obtain sufficient statistical power for the identification of new disease-causing genetic variants, as recent genome wide association studies with up to 18,000 participants have demonstrated (WTCCC 2007; Kathiresan et al. 2008; Willer et al. 2008). Moreover, by only associating genotypes with clinical outcomes, little can be inferred on the disease-causing mechanisms themselves.

Nagan *et al.* 2003 identified gene variations in SCAD, the enzyme underlying Short-chain acyl-CoA dehydrogenase deficiency. The authors found that the identified gene variations correlate with differences in C4-acylcarnitine concentrations. However, the authors came to the conclusion that none of the observed genotypes was associated with concentrations of C4-acylcarnitines that would be consistent with a biochemical diagnosis of SCAD deficiency.

Schaffer *et al.* 2006 investigated the association between SNPs in the genes encoding FADS1 and FADS2 with fatty acid composition in phospholipids. The authors analysed SNPs of the FADS1/FADS2 gene cluster, because this region was considered to be a functional and positional candidate for having a crucial influence on fatty acid composition in phospholipids and on the development of atopic diseases. They found that rare alleles of several SNPs and their respective haplotypes had a lower prevalence of allergic rhinitis in atopic eczema.

Maier *et al.* 2005 describe the association of MCAD sequence variations with distribution of metabolite concentrations in newborns with medium-chain acyl-CoA dehydrogenase deficiency (MCADD), the most frequent inherited defect of fatty acid oxidation which shows a significant morbidity and mortality in undiagnosed patients.

Döring *et al.* 2008 disclose an investigation into genetic control influences of blood uric acid concentrations as serum uric acid concentrations are correlated with gout and clinical entities such as cardiovascular disease and diabetes. The authors used a genome-wide association study to identify SNPs associated with uric acid concentrations.

Thus, a number of approaches are described in the art that aim at identifying methods to predict a predisposition for diseases based on a correlation between these diseases and genotype variations. However, research so far has only concentrated on associating genotypes with clinical outcomes and, therefore, there still exists the need for identifying genotypes that allow the identification of the metabolic capacities of individuals independently of diseases and which thus allow a prediction of individual physiological susceptibilities.

Accordingly, the present invention relates to a method for determining a predisposition of a subject for physiological susceptibilities comprising determining, in a sample obtained from the subject, the genotype of at least two genetic polymorphisms that have been identified as being associated with a variation in the metabotype of said subject, wherein the metabotype is represented by the ratio between at least two metabolites of the same biochemical pathway; and wherein the genotype of said genetic polymorphisms is indicative of a predisposition for physiological susceptibilities that result from alterations in the biochemical pathway underlying this metabotype.

The term "predisposition" in accordance with the invention refers to an increased individual risk of a subject having or developing a certain condition or disease. Such conditions or diseases include, but are not limited to, hyperactivity, a potential benefit from a specific nutrition (e.g. breast feeding and IQ) but also type 2 diabetes, metabolic syndrome, coronary artery disease, Crohn's disease, rheumatic arthritis, border line syndrome as well as levels of cholesterol and triglycerides.

The term "physiological susceptibilities" in accordance with the invention relates to a phenotype that is influenced by a disregulation of metabolic function. Preferably, said term relates to a susceptibility to disregulations of the overall fatty acid metabolism, such as for example a weakness in the fatty acid beta-oxidation, with symptoms such as hypoketotic hypoglycemia, lethargy, encephalopathy, and seizures; a lack or oversupply of polyunsaturated fatty acids (PUFA), with an impact on the prostaglandin or inflammatory pathways downstream of arachidonic acid, or an imbalance in the homeostasis of a given class of phospholipis with a modification of the lipid composition of HDL cholesterol particles and the composition of triglycerides.

The term "sample", according to the invention, refers to body fluids or tissue, preferably to blood, serum, plasma, fetal tissue, saliva, urine, mucosal tissue, mucus, vaginal tissue, fetal tissue obtained from the vagina, skin, hair, hair follicle or another human tissue. The sample may be obtained from the subject by any method known to the skilled person, including but not limited to taking cotton swabs from mouth and throat, collecting urine samples or taking blood samples.

The term "genotype" in accordance with the present invention refers to the genetic constitution of the subject with reference to genetic polymorphisms. In particular, each genetic polymorphism has two allelic forms, the minor and the major allele. The genotype describes the allelic make-up of these genetic polymorphisms, i.e. whether either the major or the minor allele is present as two copies (thus being homozygote) or whether one copy of the major allele and one copy of the minor allele is present (heterozygote).

The term "genetic polymorphism", according to the invention, refers to the occurrence of one or more different nucleotides or bases at a given location on a chromosome. Usually, genetic polymorphisms are distinguished from mutations based on their prevalence. Sometimes a threshold of at least 1% prevalence in a population of individuals is considered for separating polymorphisms (more frequent) from mutations (less frequent). Preferably, the polymorphisms have a prevalence of at least 5%, more preferably of at least 10% and most preferably, the polymorphisms have a prevalence of at least 20%. It is understood that the term "genetic polymorphism" embraces polymorphisms in exons, introns and regulatory regions such as promoters. Polymorphisms in exons may be determined or analysed using genomic DNA or cDNA (or equivalently mRNA). Polymorphisms in introns or regulatory regions such as promoters may be determined or analysed using genomic DNA.

The term "metabotype" is well established in the art and relates to the metabolic phenotype of a subject, which provides a readout of the metabolic state of an individual and is the product of genetic and environmental (diet, lifestyle, gut microbial activity) contributions. In accordance with the present invention, the metabotype is represented by the ratio between two metabolites of the same biochemical pathway. The term " metabolites of the same biochemical pathway" relates to the requirement that these metabolites belong to one pathway, i.e. that they are linked by one series of enzymatic reactions that are connected by the intermediate products wherein the reactants (or substrates) of one reaction are the products of the previous one, and so on. The term "a variation in the metabotype", as used throughout the invention, refers to differences in this metabolic make up of an individual as compared to the general metabolic phenotype of the majority of the population.

In accordance with the present invention, a genome-wide association (GWA) study with metabolic traits as phenotypic traits, using simultaneous measurements of single nucleotide polymorphisms (SNPs) and serum concentrations of endogenous organic compounds in a human population, has led to the identification of genetically determined variants in metabolic phenotype that exhibit large effect sizes. Nine of these polymorphisms are located in genes coding for well-characterized enzymes or transporters of the lipid metabolism. Thus, it was surprisingly found that individuals with different genotypes in these genes have significantly different metabolic capacities with respect to the synthesis, desaturation and elongation of some polyunsaturated fatty acids, in particular of very long chain polyunsaturated fatty acids, the beta-oxidation of short-, medium-, and long-chain fatty acids, the breakdown of triglycerides, the transport of carnitines, the synthesis of sphingolipids and of serine (a precursor of sphingolipids) as well as the activation of long-chain fatty acids for both synthesis of cellular lipids and degradation via beta-oxidation. These polymorphisms identified in accordance with the present invention all have a prevalence higher than 20% and are thus commonly found in the human population, which corresponds to a minor allele homozygote frequency that is in all cases larger than 4%. Heterozygote (carrier of one copy of the minor allele) frequencies are accordingly larger than 32%. This implies a wide applicability of the present invention, which, contrary to the findings of the prior art, is not limit to the detection of rare genetic disorders. The present invention clearly demonstrates that a GWA study with metabolomic phenotypes provides a more functional approach to the study of human genetic variation, increases the power of such studies, and allows for the identification or confirmation of new associations from previous GWA studies with clinical and non-clinical parameters as phenotypic traits.

In this approach, the concept of a *"genetically determined metabotype"* as an intermediate phenotype has been identified as a central concept, as it becomes a measurable quantity in the framework of GWA studies with metabolomics. The investigation of these genetically determined metabotypes in their biochemical context can be used to better understand gene-environment interactions as well as the pathogenesis of common diseases. These findings furthermore result in a step towards personalized health care and nutrition, based on a combination of genotyping and metabolic characterisation.

Furthermore, analyzing ratios of metabolite concentrations strongly reduces the variation in the dataset when a pair of metabolites is closely connected to the direct substrates and products of a given enzymatic reaction. When a tested genetic polymorphism impacts the efficiency of such a metabolic reaction, then the use of concentration ratios leads to drastically decreased variance and, consequentially, strongly decreased p-values of associations. Such a dependency not only provides rational evidence for a positive association, but also points to potentially affected metabolic pathways, as is demonstrated in accordance with the present invention for the example of the *FADS1* case, an observation that holds in fact for all nine SNPs of this invention. Whenever a pair of metabolites is related to the direct substrates and products of an enzymatic conversion, respectively, the ratio between their concentrations can be used as an approximation of the enzymatic activity. We thereby show that the variance in the dataset can be drastically reduced by using these ratios, which increases the power of the GWA study and reduces the corresponding p-values of association by several orders of magnitude. It was surprisingly found that by using metabolite concentration ratios, the p-value of the association with the polymorphism in the *FADS1* gene decreases by up to fourteen orders of magnitude to asymptotic p-values that were smaller than 10⁻²¹ in the initial study with 284 individual, and that gave rise to asymptotic p-values smaller than 10⁻¹⁰⁰ in a replication study with 1048 individuals. Whereas metabolite concentration ratios have been used previously, they were only used as biomarker for the identification of a rare genetic disease (e.g. newborn screen, Maier et al.). This is the first time that metabolite ratios are used without any prior hypothesis in a genome-wide scan for susceptibility genes. This method moreover assures that the identified polymorphisms are indeed located in key enzymes, as only the rate limiting steps in the lipid metabolism will be sensible to a change in enzyme or transporter activity, which is determined by metabolite ratios. This fact is confirmed in the literature for the enzymes SCAD, MCAD, LCAD, FADS1, SPTLC3, ACSL1, and PHGDH. The thereby identified metabolite pairs are found in all cases to be directly related to the proteins' functions, as represented by the red colored letters in the column "strongest metabolite pair' in Table 1, which indicate the link between the metabolite pair and the general role of the protein (e.g. the short chain lengths of the acyl carnitines C3 and C4 in the case of SCAD or the degree of saturation of the lipid side chains in the case of FADS1 - the enzyme that inserts the fourth double bond into PUFAs). The abbreviations used for the metabolites are summarized in Table 3 in the Examples section.

**Table 1. Link between the analysed metabolite pairs and the general role of the proteins in which the genetic polymorphisms are located**

| Enzyme | Strongest metabolite pair | SNPid | KEGG(Pathwayls) | General role |
|---|---|---|---|---|
| SCAD aka ACADS | C**3**/C**4** | rs2014355 | PATH:hsa00071 Fatty acid metabolism | Short chain fatty acid β-oxidation |
| | | | PATH: hsa00280 Valine, leucine and isoleucine degradation | |
| | | | PATH: hsa00650 Butanoate metabolism | |
| | | | PATH: hsa00071 Fattyacid metabolism | |
| | | | | |
| MCAD aka ACADM | C**12**/C**8** | rs11161510 | PATH: hsa00280 Valine, leucine and isoleucine degradation | chain fatty acid β-oxidation |
| | | | PATH: hsa00410 beta-Alanine metabolism | |
| | | | PATH: hsa00640 Propanoate metabolism | |
| | | | PATH: hsa03320 PPAR signaling pathway | |
| | | | | |
| LCAD aka ACADL | C9/C**14** | rs2286963 | PATH: hsa00071 Fatty acid metabolism | Long chain fatty acid β-oxidation |
| | | | PATH: hsa03320 PPAR signaling pathway | |
| | | | | |
| FADS1 | PC aa C36:**4**/ PC aa C36:**3** | rs174548 | PATH: hsa01040 Biosynthesis of unsaturated fatty acids | Synthesis of arachidonic acid |
| | | | | |
| ELOVL2 | PC aa C**40**:3/ PC aa **C42**:5 | rs9393903 | PATH: hsa01040 Biosynthesis of unsaturated fatty acids | Elongation of very long chainfatty acids |
| | | | | |
| SPTLC3 | **SM**(OH) 22:1/ **SM**(OH)24:1 | rs168622 | PATH: hsa00600 Sphingolipid metabolism | Rate limiting step in the de novo synthesis of sphingolipids |
| | | | | |
| PHGDH | Threonine/**Serine** | rs541503 | PATH: hsa00260 Glycine, serine and threonine metabolism | first and rate-limiting step in the phosphorylated pathway of serine biosynthesis |
| | | | PATH: ko00600 Sphingolipid metabolism (via Serine) | |
| ACSL1 | PC ae **C44:5/** PC ae **C42:5** PC ae **C42:5** | rs2046813 | PATH: hsa00071 Fatty acid metabolism | acyl-CoA synthetase long-chainfamily; preferentially uses palmitoleate, oleate and linoleate |
| | | | PATH: hsa03320 PPAR signaling pathway | |
| | | | PATH: hsa04920 Adipocytokine signaling pathway | |
| OCTN1 | Valine/C5 | rs272889 | PATH: this transporter is not described by a KEGG pathway; PATH: this transporter is not described by a KEGG pathway; It is involved in the acyl carnitine metabolism | sodium-ion dependent, low affinity carnitine transporter (SLC22A4) |

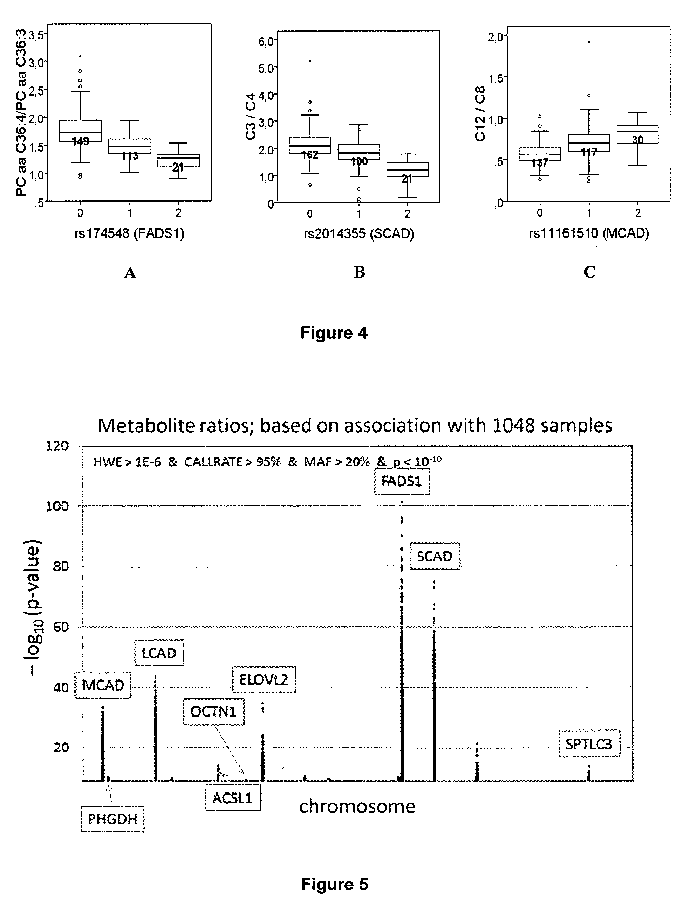

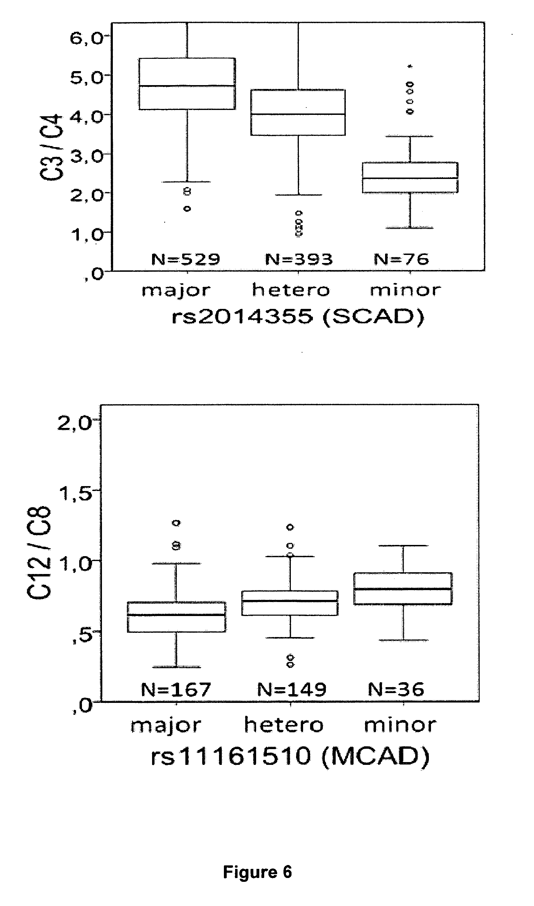

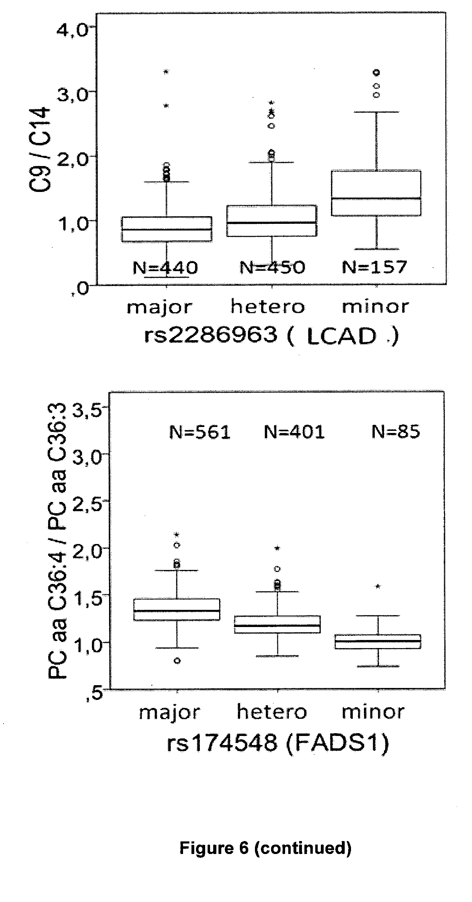

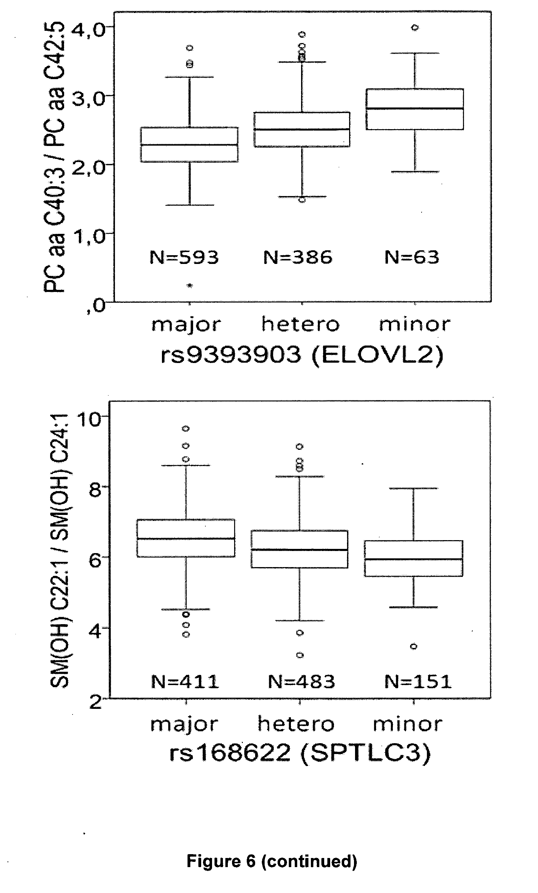

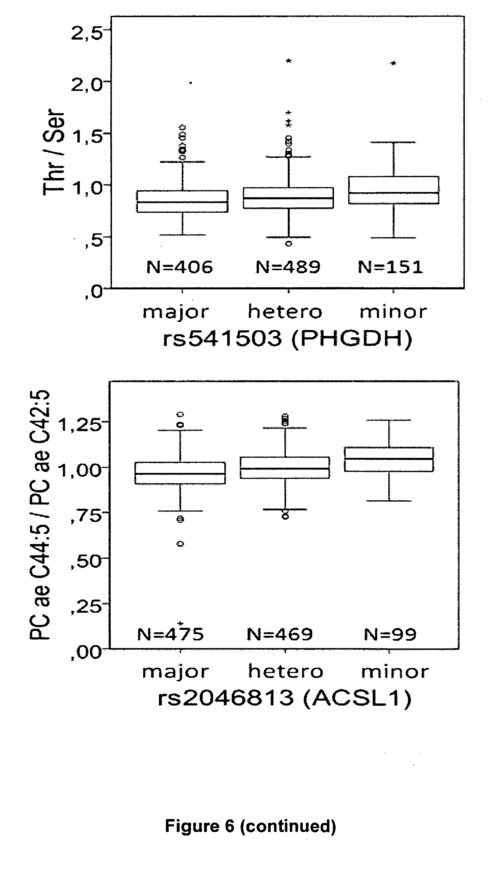

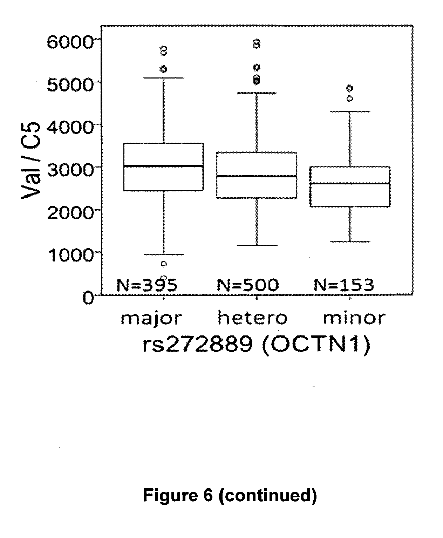

Furthermore, as opposed to standard approaches used in this field, the approach underlying the present invention has not been based on any prior hypothesis as to which genes should be screened for a possible association with metabolite ratios. As can be seen from Figure 5, the association between key proteins of the lipid metabolism and the strongest matching metabolite pairs (Figure 6 and Table 1) are automatically identified without any prior hypothesis. For instance, if the molecular function of FADS1 had not been already known, the association between the SNP and the different glycerophospholipid concentrations per se would have allowed to deduce its enzymatic activity of inserting a fourth double bond into long-chain fatty acids. Thus, by using this unique approach it was possible to detect the key proteins of the lipid metabolism that exist in two differently active, or differently regulated, versions in the human inheritance. The method of this invention is based on this discovery and allows to "visualize" the metabolic capabilities of any given individual.

In a preferred embodiment of the method of the invention, the association of a genetic polymorphism with a variation in the metabotype has been identified by the following steps: a) determining the concentration of metabolites in samples obtained from a group of individuals; b) providing a ratio between at least two of these metabolites, wherein the metabolites are of the same biochemical pathway; c) determining genetic polymorphisms in samples obtained of this group of individuals; and d) associating the genetic polymorphisms identified in (c) with variations in the ratio between the at least two metabolites obtained in (b).

As shown in the appended examples and as discussed above, it was found in the context of the present invention that by determining the concentration of metabolites in samples obtained from a group of individuals and concurrently determining genetic polymorphisms in samples obtained from the same group of individuals, genetic polymorphisms can be identified that are associated with variations observed in the metabolite concentrations between different individuals. Furthermore, whenever a pair of metabolites is related to the direct substrates and products of an enzymatic conversion, respectively, the ratio between their concentrations can be used as an approximation of the enzymatic activity. It was thereby shown in accordance with the present invention that the variance in the dataset can be drastically reduced by using these ratios, which increases the power of the GWA study and reduces the corresponding p-values of association by several orders of magnitude.

In a further preferred embodiment, the step of associating genetic polymorphisms with metabolite ractios in (d) is carried out using a genome-wide association study.

In an alternative or a preferred embodiment, the invention relates to a method for determining a predisposition of a human subject for physiological susceptibilities that result from alterations in lipid metabolism, comprising determining, in a sample obtained from the human subject, the genotype of that person with respect to at least two genetic polymorphisms selected from the group consisting of a) rs2014355; wherein the minor allele is represented by a cytidine and the major allele is represented by a thymidine; b) rs11161510; wherein the minor allele is represented by a thymidine and the major allele is represented by a cytidine; c) rs2286963; wherein the minor allele is represented by a guanosine and the major allele is represented by a thymidine; d) rs174548; wherein the minor allele is represented by a guanosine and the major allele is represented by a cytidine; e) rs9393903; wherein the minor allele is represented by a adenosine and the major allele is represented by a guanosine; f) rs168622; wherein the minor allele is represented by a thymidine and the major allele is represented by a guanosine; g) rs541503; wherein the minor allele is represented by a cytidine and the major allele is represented by a thymidine; h) rs2046813; wherein the minor allele is represented by a cytidine and the major allele is represented by a thymidine; i) rs272889; wherein the minor allele is represented by a adenosine and the major allele is represented by a guanosine; and j) at least one genetic polymorphism that is in linkage disequilibrium with any of the genetic polymorphisms of (a) to (i), wherein the presence of one or two copies of the minor allele of the at least two genetic polymorphisms is indicative of a predisposition for said physiological susceptibilities.

This embodiment, on one hand, represents a preferred embodiment of the method of the invention as disclosed above. On the other hand, this embodiment can also be considered an alternative, i.e. independent, embodiment of a method for determining a predisposition of a human subject for physiological susceptibilities that result from alterations in lipid metabolism.

The term "genotype" has been defined above. The skilled person readily understands that in order to determine the genotype of a particular genetic polymorphism, the presence of a major allele or of a minor allele on both sister chromosomes has to be investigated. It is then immediately possible to deduce whether the genetic polymorphism is homozygous for either the major or minor allele or whether it is heterozygous (i.e. possesses one copy of the major and one copy of the minor allele). The contribution of the alleles to the phenotype is additive, i.e. the presence of two minor alleles is indicative of variations in the metabolic make-up of the subject that are more severe than variations associated with merely one copy of the minor allele.

The term "rs2014355" relates to the genetic polymorphism rs2014355 located on human chromosome 12, in position 119659907 of the (+) strand (according to the NCBI build 36.3). In this genetic polymorphism the thymidine (T), that is present in the majority of people, and thus is the major allele, is replaced by a cytosine (C), which represents the minor allele. The same applies accordingly to the genetic polymorphisms defined in (b) to (i), wherein the location of said genetic polymorphisms, the minor and major alleles, the surrounding sequence as well as the enzyme encoded by the affected gene are as summarised in Table 2. The identification of the minor and major alleles as used herein is always based on the (+) strand of the DNA sequence, as shown in Table 2.

The skilled person understands that also the determination of the corresponding nucleotide in the opposite strand may be used for determining the genotype of a genetic polymorphisms in accordance with the method of the invention. The corresponding nucleotides are cytosine where the (+) strand contains a guanosine and correspondingly a guanosine when the (+) strand contains a cytosine. If the (+) strand contains a thymidine the corresponding nucleotide is an adenosine and correspondingly it is a thymidine when the (+) strand contains an adenosine.

The term "Linkage Disequilibrium" in accordance with the present invention refers to the phenomenon that the DNA sequences which are close together in the genome have a tendency to be inherited together. Two or more sequences may be linked because of some selective advantage of co-inheritance. More typically, however, two or more polymorphic sequences are co-inherited because of the relative infrequency with which meiotic recombination events occur within the region between the two polymorphisms. The co-inherited polymorphic alleles are said to be in linkage disequilibrium with one another because, in a given population, they tend to either both occur together or else not occur at all in any particular member of the population. Indeed, where multiple polymorphisms in a given chromosomal region are found to be in linkage disequilibrium with one another, they define a quasi-stable genetic "haplotype." Furthermore, where a phenotype-causing mutation is found within or in linkage with this haplotype, one or more polymorphic alleles of the haplotype can be used as a diagnostic or prognostic indicator of the likelihood of developing a specific phenotype. Identification of a haplotype which spans or is linked to a phenotype-causing mutational change, serves as a predictive measure of an individual's likelihood of having inherited that phenotype-causing mutation. Importantly, such prognostic or diagnostic procedures can be utilized without necessitating the identification and isolation of the actual phenotype-causing molecule. This is significant because the precise determination of the molecular basis of the establishment of a specific phenotype can be difficult and laborious, especially in the case of multifactorial phenotype.

As discussed above, several genetically determined variants in metabolic phenotype that exhibit large effect sizes have been found in accordance with the present invention. The nine genetic polymorphisms of this preferred embodiment are located in genes coding for well-characterized enzymes or transporters of the lipid metabolism. Thus, it was surprisingly found that individuals with different genotypes in these genes have significantly different metabolic capacities with respect to the synthesis, desaturation and elongation of some polyunsaturated fatty acids, in particular of very long chain polyunsaturated fatty acids, the beta-oxidation of short-, medium-, and long-chain fatty acids, the breakdown of triglycerides, the transport of carnitines, the synthesis of sphingolipids and of serine (a precursor of sphingolipids) as well as the activation of long-chain fatty acids for both synthesis of cellular lipids and degradation via beta-oxidation.

Due to the abundance of established methods for assessing the genotypes of genetic polymorphisms, the present invention now allows for a convenient prediction of a predisposition for physiological susceptibilities that result from alterations in lipid metabolism in a short amount of time, at low cost, with high accuracy and without significant trouble for the person under investigation.

**Table 2: The location of the genetic polymorphisms (according to the NCBI build 36.3), their surrounding DNA sequence at this chromosomal location, their respective minor and major alleles, as well as the enzymes encoded by the affected genes.**

| SNP ID | Chromosomal location | Sequence | Minor allele | Major allele | Enzyme |
|---|---|---|---|---|---|
| rs2014355 | Chr.12: 119659907(+) | TGGGACCCTCA TCTTT/CGGAG CCCGAGTCATA | C | T | Intronic SNP in SCAD aka ACADS: acyl-Coenzyme A dehydrogenase, C-2 to C-3 short chain (EC 1.3.99.2); also ACAD3: Butyryl-CoA dehydrogenase |
| | | | | | |
| rs1116151 0 | Chr1: 75982877(+) | GTAGTTCTGAA GAAGC/TGAAC ACTGAAGACAC | T | C | Intronic SNP in MCAD aka ACADM: acyl-Coenzyme A dehydrogenase, C-4 to C-12 straight chain (EC 1.3.99.3); also ACAD1: medium-chain specific acyl-CoA dehydrogenase |
| | | | | | |
| rs2286963 | Chr 2: 210768295(+) | TTAATTCTGCT AATTG/TATGT TGCACTGTCTT | G | T | Coding SNP in LCAD aka ACADL: acyl-Coenzyme A dehydrogenase, long chain (EC:1.3.99.13 and EC:1.3.99.3); also ACAD4: long-chain specific acyl-CoA dehydrogenase |
| | | | | | |
| rs174548 | Chr 11: 61327924(+) | GGTTATCCAGA CTCAC/GTCAT CTTCAGCTTCT | G | C | Intronic SNP in FADS1: fatty acid desaturase 1 (EC:1.14.99.25); also D5D: Delta(5) fatty acid desaturase |
| | | | | | |
| rs9393903 | Chr 6: 11150895(+) | GCATAAGAGAT AGCCA/GCCTA GACTCGTCATT | A | G | Intronic SNP in ELOVL2: elongation of very long chain fatty acids (FEN1/Elo2, SUR4/Elo3, yeast)-like 2 (EC:2.3.1.-) |
| | | | | | |
| rs168622 | Chr 20: 12914089(+) | GGTCAGGAGAA ACCAT/GTTCT AACCCCAGCTC | T | G | SNP upstream of and in LD with SPTLC3: serine palmitoyltransferase, long chain base subunit 3 (EC:2.3.1.50); |
| | | | | | |
| rs541503 | Chr1: 120009820(+) | AAACTGGTAAA GTCCC/TGGAT ACAAGAGGCTC | C | T | SNP upstream of and in LD with PHGDH: phosphoglycerate dehydrogenase (EC 1.1.1.95) |
| | | | | | |
| rs2046813 | Chr 4: 186006153 (+) | GATGTGTGAAG TTTAC/TTATG TGCAAAAACCT | C | T | SNP upstream of ACSL1: acyl-CoA synthetase long-chain family member 1 (EC:6.2.1.3); |
| | | | | | |
| rs272889 | Chr5: 131693277(+) | TATTGTCTTAG AAAGCA/GATC TCAAGCATCCT | A | G | Intronic SNP in SLC22A4: solute carrier family 22 (organic cation / ergothioneine transporter), member 4; also OCTN 1: Organic cation/carnitine transporter 1 |

In a further preferred embodiment, the human subject is of kaukasian descent.

In another preferred embodiment of the method of the invention, the presence of one or two copies of the minor allele of a) rs2014355 or a genetic polymorphism in linkage disequilibrium thereto is associated with a decreased yield of the enzymatic reactions that are catalyzed by SCAD (EC:1.3.99.2); b) rs11161510 or a genetic polymorphism in linkage disequilibrium thereto is associated with a decreased yield of the enzymatic reactions that are catalyzed by MCAD (EC:1.3.99.3); c) rs2286963 or a genetic polymorphism in linkage disequilibrium thereto is associated with an increased yield of the enzymatic reactions that are catalyzed by LCAD (EC:1.3.99.13 and EC:1.3.99.3); d) rs174548 or a genetic polymorphism in linkage disequilibrium thereto is associated with a decreased yield of the enzymatic reactions that are catalyzed by FADS1 (EC:1.14.99.25); e) rs9393903 or a genetic polymorphism in linkage disequilibrium thereto is associated with a decreased yield of the enzymatic reactions that are catalyzed by ELOVL2 (EC:2.3.1.-); f) rs168622 or a genetic polymorphism in linkage disequilibrium thereto is associated with an increased affinity for longer chain sphingomyelins of the regulatory subunit SPTLC3 in the SPT enzymatic complex (EC:2.3.1.50); g) rs541503 or a genetic polymorphism in linkage disequilibrium thereto is associated with a increased yield of the enzymatic reaction that is catalyzed by PHGDH (EC:1.1.1.95); h) rs2046813 or a genetic polymorphism in linkage disequilibrium thereto is associated with an increased substrate affinity for longer chain fatty acids of the enzymatic reactions that are catalyzed by ACSL1 (EC:6.2.1.3); i) rs272889 or a genetic polymorphism in linkage disequilibrium thereto is associated with an increased transporter activity of C5-Acylcarnitine that is catalyzed by OCTN1.

The term "the presence of one or two copies of the minor allele" as used in accordance with the present invention, relates to the heterozyous (one copy) or homozygous (two copies) presence of the minor allele of the investigated genetic polymorphism. As discussed above, the presence of the minor allele associates in an additive manner with the effect observed on the metabotype. This means that the presence of two copies of the minor allele (i.e. a homozygote subject) is associated with an alteration in the lipid metabolism that is twice as strong as the effect associated with the presence of one copy of the minor allele (i.e. in a heterozygote subject), wherein subjects that are homozygous for the major allele represent a phenotype that is consistent with the majority of the population .

The term "a decreased yield of the enzymatic reactions that are catalyzed by SCAD (EC:1.3.99.2)" in accordance with the present invention relates to a slower degradation of short-chain fatty acids (up to C6) in the beta-oxidation pathway, and more specifically to the acetyl-CoA dehydrogenase reaction that initiates this pathway. As the beta-oxidation represents the central energy source of the body under starvation conditions, a decreased yield of the enzymatic reactions that are catalyzed by SCAD is expected to be associated in situations of prolonged starvation or physical activity with hypoglycemia. Such individuals may then display the corresponding symptoms, such as tiredness, loss of alertness, headache, and memory problems, or in more extreme cases, systemic disorders with clinical symptoms such as hypoketotic hypoglycemia, lethargy, encephalopathy, and seizures.

The term "a decreased yield of the enzymatic reactions that are catalyzed by MCAD (EC:1.3.99.3)" in accordance with the present invention relates to a slower degradation of medium-chain fatty acids (preferentially C4 to C12) in the beta-oxidation pathway, and more specifically to the acetyl-CoA dehydrogenase reaction that initiates this pathway.
As the beta-oxidation represents the central energy source of the body under starvation conditions, a decreased yield of the enzymatic reactions that are catalyzed by MCAD is expected to be associated in situations of prolonged starvation or physical activity with hypoglycemia. Such individuals may then display the corresponding symptoms, such as tiredness, loss of alertness, headache, and memory problems, or in more extreme cases, systemic disorders with clinical symptoms such as hypoketotic hypoglycemia, lethargy, encephalopathy, and seizures.

The term "an increased yield of the enzymatic reactions that are catalyzed by LCAD (EC:1.3.99.13 and EC:1.3.99.3)" in accordance with the present invention relates to a slower degradation of long-chain fatty acids (preferentially C6 to C16) in the beta-oxidation pathway, and more specifically to the acetyl-CoA dehydrogenase reaction that initiates this pathway.

As the beta-oxidation represents the central energy source of the body under starvation conditions, a decreased yield of the enzymatic reactions that are catalyzed by LCAD is expected to be associated in situations of prolonged starvation or physical activity with hypoglycemia. Such individuals may then display the corresponding symptoms, such as tiredness, loss of alertness, headache, and memory problems, or in more extreme cases, systemic disorders with clinical symptoms such as hypoketotic hypoglycemia, lethargy, encephalopathy, and seizures.

The term " a decreased yield of the enzymatic reactions that are catalyzed by FADS1 (EC:1.14.99.25)" in accordance with the present invention relates to a slower conversion of eicosatrienoyl-CoA (C20:3-CoA) to arachidonyl-CoA (C20:4-CoA) by the delta-5 fatty acid desaturase reaction.

Arachidonic acid (C20:4) is a central metabolite in the the polyunsaturated fatty acid pathway (PUFA). PUFA composition in phospholipids has been shown to be associated with the outcome of several complex human diseases such as the metabolic syndrome, cardiovascular diseases, psychiatric disorders and immune-related diseases such as chronic obstructive pulmonary disease and osteoarthritis. Fatty acids have been suggested to play a major role in the development of allergies, as PUFAs are processed to powerful promoters of inflammation called eicosanoids such as prostaglandins and leukotrienes. PUFA levels in phospholipids are known to be determined by both nutrition and the metabolism. A decreased yield of the enzymatic reactions that are catalyzed by FADS1 is therefore expected to disturb the effect of especially designed functional foods, diets or other nutritional treatments by limiting the production in the body of PUFAs beyond C20:3 and also by accumulating smaller PUFAs accordingly. An imbalance in the homeostasis of PUFAs is also expected to change the homeostasis of certain classes of phospholipis (PC, PE, PI, with ester and ether bonds) with the consequence of a modification of the lipid composition of HDL cholesterol particles and the composition of triglycerides. There is evidence for a link between the long-chain polyunsaturated fatty acid metabolism and attention deficit/hyperactivity syndrome (ADHS). Genetic variation in the FADS gene cluster has also been shown to moderate the association between breastfeeding and intelligence quotient (IQ), by influencing the ability to metabolize certain fatty acids that are uniquely available in breast milk.

The term "a decreased yield of the enzymatic reactions that are catalyzed by ELOVL2 (EC:2.3.1-)" in accordance with the present invention relates to a slower elongation of the omega-6 fatty acids C20:4 to C22:4 and C22:4 to C24:4 and of the omega-3 fatty acids C20:5 to C22:5 and C22:5 to C24:5.

PUFA composition in phospholipids has been shown to be associated with the outcome of several complex human diseases such as the metabolic syndrome, cardiovascular diseases, psychiatric disorders and immune-related diseases such as chronic obstructive pulmonary disease and osteoarthritis. Fatty acids have been suggested to play a major role in the development of allergies, as PUFAs are processed to powerful promoters of inflammation called eicosanoids such as prostaglandins and leukotrienes. PUFA levels in phospholipids are known to be determined by both nutrition and the metabolism.

The term "an increased affinity for longer chain sphingomyelins of the regulatory subunit SPTLC3 in the SPT enzymatic complex (EC:2.3.1.50)" in accordance with the present invention relates to the concept that enzymatic reactions are often found to be endproduct-regulated, as this is the case here. The initial and rate limiting step of the sphingolipid synthesis is negatively regulated by the concentration of sphingolipids themselves. The regulatory function of the SPT complex is implemented by SPTLC3. The sensibility of SPTLC3 to regulation by sphingolipids differs by sphingolipid chain length. In accordance with the present invention, SPTLC3 is more sensitive to regulation by longer chain sphingolipids, i.e. it shows an increased affinity for longer chain sphingolipids.

The result of such a regulatory difference is a change in sphingolipid composition. Sphingolipids are, together with phospholipids and cholesterol the major constituents of cell membranes, including cell membranes of neuronal cell (hence the name sphingolipids). Lipid lowering drugs, such as statins, may interact with sphingolipid composition. Insulin has also been shown to impact on sphingolipid composition. It can thus be expected that a polymorphism in SPTLC3 can impact the outcome of the testing and application of lipid-targeting drugs..

The term "an increased yield of the enzymatic reaction that is catalyzed by PHGDH (EC:1.1.1.95)" in accordance with the present invention relates to a higher production of serine in the first and rate-limiting step in the phosphorylated pathway of serine biosynthesis by the phosphoglycerate dehydrogenase reaction.
Serine is used as a substrate in the sphingolipids biosynthesis and thus, the above discussed implications also apply here.

The term "an increased substrate affinity for longer chain fatty acids of the enzymatic reactions that are catalyzed by ACSL1 (EC:6.2.1.3)" in accordance with the present invention relates to a change in preference for free long-chain fatty acids in the conversion into fatty acyl-CoA esters. Thereby this reaction plays a key role in lipid biosynthesis and fatty acid degradation.

The term "an increased transporter activity of C5-Acylcarnitine that is catalyzed by OCTN1" in accordance with the present invention relates to increases in the transport efficiency or substrate specificity of this protein, that has been described as a member of a familily of polyspecific organic cation transporter in the liver, kidney, intestine, and other organs and that is critical for elimination of many endogenous small organic cations as well as a wide array of drugs and environmental toxins.
In accordance with the present invention, in particular acylcarnitine C5 is impacted by an increased transporter activity that is catalyzed by OCTN1. Variants of the OCTN1 (also known as SLC22A4) gene are associated with susceptibility to rheumatoid arthritis and Crohn's disease.

The above described enzymes have been classified according to the EC numbering system, which is the common system for enzyme classification used in the art. The Enzyme Commission number (EC number) is a numerical classification scheme for enzymes, based on the chemical reactions they catalyze. As a system of enzyme nomenclature, every EC number is associated with a recommended name for the respective enzyme. Every enzyme code consists of the letters "EC" followed by four numbers separated by periods. Those numbers represent a progressively finer classification of the enzyme. The first number defines the enzyme class. In general, enzymes are divided into six different enzyme classes: Oxidoreductases, Transferases, Hydrolases, Lyases, Isomerases and Ligases. The further numbers provide a more detailed characterisation of the enzymes, such as for example information about the substrate, the group that is transferred or the kind of bond that is formed or cleaved.

In accordance with the present invention it was found in one aspect that frequent genetically determined metabotypes play a role as discriminating cofactors in the etiology of common multi-factorial diseases. In interactions with environmental factors such as nutrition or life style, these metabotypes may influence the susceptibility of an individual for certain phenotypes. As an example, there is growing evidence for a link between the long-chain polyunsaturated fatty acid metabolism and attention deficit/hyperactivity syndrome (ADHS). An association of the same polymorphism in the FADS1 gene that was identified in accordance with the present invention (rs174548) has recently been reported to be associated with ADHS (Brookes et al. 2006). Genetic variation in the FADS gene cluster has also been shown to moderate the association between breastfeeding and intelligence quotient (IQ), by influencing the ability to metabolize certain fatty acids that are uniquely available in breast milk (Caspi et al. 2007). Such effects may possibly be explained by changes in the membrane fluidity of neuronal cells, which depends on the degree of membrane fatty acid saturation, and consequentially impacts the mobility of membrane-bound neuroreceptors. The differentiation of the population into individuals with different levels of four-fold and higher-fold unsaturated fatty acids, as induced by the FADS1 polymorphism, is thus a prototype of a genetically determined metabotype.

Of particular interest are also the two polymorphisms in the SCAD and MCAD genes. Major deficiencies in the corresponding enzymes are known to be associated with severe systemic disorders and with clinical symptoms such as hypoketotic hypoglycemia, lethargy, encephalopathy, and seizures. Such deficiencies are nowadays systematically identified by neonatal screening programs (Maier et al. 2005 and references therein). In contrast, the genetic variants of SCAD and MCAD genes that were found in accordance with the present invention show a relatively moderate phenotypic expression, but are very frequent in the population (minor allele frequencies > 25%). Individuals that are homozygous for at least one of the minor alleles of the SCAD or MCAD polymorphisms are likely to show signs of impaired beta-oxidation. One would expect that, for instance in situations of prolonged starvation or physical activity, these individuals may become more readily hypoglycemic and may display the corresponding symptoms, such as tiredness, loss of alertness, headache, and memory problems. It is therefore promising to search for associations between the SCAD/MCAD polymorphisms and phenotypes that are related to impaired beta-oxidation, either in the context of diabetes or in the context of physical activity.

The identification of genetic variants that alter the homeostasis of key metabolites in the human body will eventually lead to a functional understanding of the genetics of complex diseases. Progress towards individualized medication lies in a combination of genotyping and metabotyping, as shown in the context of the present invention. It is concluded that metabolomics delivers its promise of providing access to functionally relevant endpoints in the framework of GWA studies, and thereby opens new avenues for a functional investigation of the role of gene-environment interactions in the etiology of complex diseases.

In a further preferred embodiment of the method of the invention, the genotype of at least three genetic polymorphisms is determined.

Furhter preferrerd in accordance with the present invention is that the genotype of at least four, at least five, at least six, at least seven, at least eight or at least nine genetic polymorphisms is determined.

In another preferred embodiment of the method of the invention, the genotype of at least one genetic polymorphism for each of the identified pathways is determined. Thus, the genotype of at least one of a) rs2014355 (SCAD), rs1116151 (MCAD) or rs2286963 (LCAD) is determined in order to investigate the beta-oxidation of fatty acids; b) rs174548 (FADS1) or rs9393903 (ELOVL2) is determined in order to investigate the metabolism of very long chain PUFAs; and c) rs168622 (SPTLC3) or rs541503 (PHGDH) is determined in order to investigate the metabolism of sphingolipids.

As discussed above, the approach that lead to identification of the method of the present invention is unique in the field in that it has not been based on any prior hypothesis as to which genes should be screened for a possible association with metabolite ratios. By using this unique approach it was possible to detect proteins that play an essential role in the key pathways of lipid metabolism. By determining the genotype of at least one genetic polymorphism for each of these identified key pathways, it is now possible to perform a comprehensive investigation of the relevant metabolic pathways of an individual and to predict the particular metabolic capabilities of this individual.

In another preferred embodiment of the method of the invention, the presence of at least the genetic polymorphisms of (a) to (i) is determined.

By determining the genotype of all the genetic polymorphisms identified in the context of the present invention as being key enzymes of the lipid metabolism it is furthermore now possible to perform a comprehensive and extensive investigation of the metabolic make-up of an individual and, thus, to predict the particular metabolic capabilities of this individual.

In a further preferred embodiment of the method of the invention, the genetic polymorphisms are selected from the group consisting of single nucleotide polymorphisms, insertions or deletions.

The term "single nucleotide polymorphism (SNP)" in accordance with the present invention refers to a DNA sequence variation occurring when a single nucleotide - A, T, C, or G - in the genome differs between paired chromosomes in an individual or between members of a species. Almost all common SNPs have only two alleles. For a variation to be considered a SNP, it must occur in at least 1% of the population. Within a population, SNPs can be assigned a minor allele frequency, which is the lowest allele frequency at a locus that is observed in a particular population. Single nucleotide polymorphisms may fall within coding sequences of genes, non-coding regions of genes, or in the intergenic regions between genes. SNPs within a coding sequence will not necessarily change the amino acid sequence of the protein that is produced, due to degeneracy of the genetic code. A SNP in which both forms lead to the same polypeptide sequence is termed *synonymous* or a silent mutation. If a different polypeptide sequence is produced they are *non-synonymous.* SNPs that are not in protein-coding regions nonetheless can affect gene splicing, transcription factor binding, or the sequence of non-coding RNA.

The term "insertion" in accordance with the present invention refers to the addition of one or more nucleotides to a nucleic acid molecule, wherein the addition is not to the 5' or 3' end of the nucleic acid molecule.

The term "deletion" as used in accordance with the present invention refers to the loss of nucleotides.

In another preferred embodiment of the method of the invention, the genotype of the genetic polymorphisms is detected by PCR based techniques, DNA sequencing-based techniques, hybridization-based techniques, single-strand conformation polymorphism analysis (SSCA), denaturating gradient gel electrophoresis (DGGE), mismatch cleavage detection, heteroduplex analysis, primer extension-based techniques, or 5'-nuclease assay-based techniques. Said techniques are well known to the person skilled in the art.

Non-limiting examples for nucleic acid amplification assays and means to perform such include PCR, (including nested PCR, RT-PCR, quantitative real-time detection, PCR extension assays, Nucleic Acid Sequence Base Amplification (NASBA), single-strand confirmation polymorphism (SSCP) PCR, PCR-restriction enzyme fragment length polymorphism (RFLP) analysis), amplification refractory mutation systems (ARMSTM) and amplification refractory mutation system linear extension (ALEXTM) assays. Details of such methods can be found in art, see, for example, Newton et al., Nucleic Acids Res. 17 (1989) 2503-2516; Agrawal (Ed.), "Protocols for Oligonucleotides and Analogs: Synthesis and Properties (Methods in Molecular Biology, 20)", Humana Press, 1993; Haque et al., Diagn. Mol. Pathol. 7 (1998) 248-252; Innis et al. (Ed.), "PCR Applications: Protocols for Functional Genomics", Academic Press, 1999; Chen and Janes (Ed.), "PCR Cloning Protocols: From Molecular Cloning to Genetic", 2nd edition, Humana Press, 2002; Pissard et al., Clin. Chem. 48 (2002) 769-772; Blondal et al., Nucleic Acids Res 31 (2003) e155; Steemers et al., Nature Meth. 3 (2006) 31-33; Kakavas et al., J. Clin. Lab. Anal. 20 (2006) 1-7.

Examples for sequencing assays comprise without limitation approaches of sequence analysis by direct sequencing, fluorescent SSCP in an automated DNA sequencer and Pyrosequencing. These procedures are common in the art, see e.g. Adams et al. (Ed.), "Automated DNA Sequencing and Analysis", Academic Press, 1994; Alphey, "DNA Sequencing: From Experimental Methods to Bioinformatics", Springer Verlag Publishing, 1997; Ramon et al., J. Transl. Med. 1 (2003) 9; Meng et al., J. Clin. Endocrinol. Metab. 90 (2005) 3419-3422.

Examples for hybridization assays comprise without limitation Northern and Southern blot assays, heteroduplex analysis, detection of mutations by sequence specific oligonucleotide hybridization, allele-specific oligonucleotide hybridization on DNA chips, assays based on Illumina's^{®} technology, assays based on the BeadArray^{®} technology, see, for example, Barnes et al., Nucleic Acids Res. 33 (2005) 5914-5923; Fan et al., Biotechniques 39 (2005) 583-588; Shen et al., Mutat. Res.-Fund. Mol. M. 573 (2005) 70-82; Steemers and Gunderson, Pharmacogenomics, 6 (2005) 777-782.

The term "hybridises/hybridising" as used herein refers to a pairing of a nucleic acid molecule to a (partially) complementary strand of this nucleic acid molecule which thereby form a hybrid.

It is well known in the art how to perform hybridization experiments with nucleic acid molecules. Correspondingly, the person skilled in the art knows what hybridization conditions she/he has to use to allow for a successful hybridization. The establishment of suitable hybridization conditions is referred to in standard text books such as Sambrook, Russell "Molecular Cloning, A Laboratory Manual", Cold Spring Harbor Laboratory, N.Y. (2001); Ausubel, "Current Protocols in Molecular Biology", Green Publishing Associates and Wiley Interscience, N.Y. (1989), or Higgins and Hames (Eds.) "Nucleic acid hybridization, a practical approach" IRL Press Oxford, Washington DC, (1985).

Appropriate stringent hybridization conditions for each nucleic acid sequence may be established by a person skilled in the art on well-known parameters such as temperature, composition and length of the nucleic acid molecules, salt conditions etc.; see, for example, Sambrook et al., "Molecular Cloning, A Laboratory Manual"; CSH Press, Cold Spring Harbor, 1989 or Higgins and Hames (eds.), loc. cit. , see in particular the chapter "Hybridization Strategy" by Britten & Davidson, 3 to 15. Such conditions comprise, e.g. an overnight incubation at 65°C in 4x SSC (600 mM NaCl, 60 mM sodium citrate) followed by washing at 65°C in 0.1x SSC for one hour. Alternatively, hybridization conditions can comprise: an overnight incubation at 42°C in a solution comprising 50% formamide, 5x SSC (750 mM NaCl, 75 mM sodium citrate), 50 mM sodium phosphate (pH 7.6), 5x Denhardt's solution, 10% dextran sulphate, and 20 µg/ml denatured, sheared salmon sperm DNA, followed by washing in e.g. 0.1-0.5x SSC at about 55-65°C for about 5 to 20 min. Changes in the stringency of hybridization are primarily accomplished through the manipulation of formamide concentration (lower percentages of formamide result in lowered stringency), salt conditions, or temperature. Based on the above discussed variables composition and length of the nucleic acid molecules, temperature, salt concentrations etc., the skilled person knows how to determine suitably stringent hybridization conditions capable of specifically detecting even single base pair mismatches. It is of note that variations in the above conditions may be accomplished through the inclusion and/or substitution of alternate blocking reagents. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility. Such modifications can generally be effected by the skilled person without further ado.

A hybridization complex may be formed in solution (e.g., Cot or Rot analysis) or between one nucleic acid sequence present in solution and another nucleic acid sequence immobilized on a solid support (e.g., membranes, filters, chips, pins or glass slides).

It is also envisaged herein that the genotype of the genetic polymorphism is determined using antibodies or sequence-specific DNA-binding proteins.

Antibodies, in accordance with the present invention, can be for example, polyclonal or monoclonal. The term "antibody" also comprises derivatives or fragments thereof which still retain the binding specificity. Techniques for the production of antibodies are well known in the art and described, e.g. in Harlow and Lane "Antibodies, A Laboratory Manual", Cold Spring Harbor Laboratory Press, 1988 and Harlow and Lane "Using Antibodies: A Laboratory Manual" Cold Spring Harbor Laboratory Press, 1999.
The antibody described in the context of the invention is capable to specifically bind/interact with the genetic polymorphisms of the invention. The term "specifically binding/interacting with" as used in accordance with the present invention means that the antibody does not or essentially does not cross-react with a similar sequence. Thus, if the antibody specificially binds the minor allele, then it does not bind to the major allele of a genetic polymorphism of the present invention. Cross-reactivity of a panel of antibodies under investigation may be tested, for example, by assessing binding of said panel of antibodies under conventional conditions to the sequence of interest as well as to a number of more or less closely related sequences. Only those antibodies that bind to the sequence of interest in its relevant context but do not or do not essentially bind to any of the other sequences are considered specific for the sequence of interest and thus to be antibodies in accordance with this invention.

The antibody of the invention also includes embodiments such as chimeric (human constant domain, non-human variable domain), single chain and humanized (human antibody with the exception of non-human CDRs) antibodies, as well as antibody fragments, like, inter alia, Fab or Fab' fragments. Antibody fragments or derivatives further comprise Fd, F(ab')₂, Fv or scFv fragments; see, for example, Harlow and Lane (1988) and (1999), loc. cit. Various procedures are known in the art and may be used for the production of such antibodies and/or fragments. Thus, the (antibody) derivatives can be produced by peptidomimetics. Further, techniques described for the production of single chain antibodies (see, inter alia, US Patent 4,946,778) can be adapted to produce single chain antibodies specific for polypeptide(s) and fusion proteins of this invention. Also, transgenic animals or plants (see, e.g., US patent 6,080,560) may be used to express (humanized) antibodies specific for the target of this invention. Most preferably, the antibody of this invention is a monoclonal antibody. For the preparation of monoclonal antibodies, any technique which provides antibodies produced by continuous cell line cultures can be used. Examples for such techniques include the hybridoma technique (Köhler and Milstein Nature 256 (1975), 495-497), the trioma technique, the human B-cell hybridoma technique (Kozbor, Immunology Today 4 (1983), 72) and the EBV-hybridoma technique to produce human monoclonal antibodies (Cole et al., Monoclonal Antibodies and Cancer Therapy, Alan R. Liss, Inc. (1985), 77-96). It is also envisaged in the context of this invention that the term "antibody" comprises antibody constructs which may be expressed in cells, e.g. antibody constructs which may be transfected and/or transduced via, amongst others, viruses or plasmid vectors.

The term "sequence-specific DNA-binding proteins" in accordance with the present invention relates to proteins capable of specifically binding to individual DNA sequences, such as for example the genetic polymorphisms of the invention. Examples of such sequence-specific DNA-binding proteins are zinc finger proteins, that can be modified to recognise specific DNA sequences.

In another preferred embodiment of the method of the invention, the genetic polymorphisms are detected using a solid phase support.

The term "solid phase support" is well known to the skilled person. Non-limiting examples of a solid phase support are beads, chips and membranes.

In a preferred embodiment, the solid phase support is a DNA chip. Methods for the production of DNA chips as well as the use of DNA chip technology are well known in the art and described e.g. in Lohara et al., Nucleic Acids Res. 30 (2002) e87; Flavell et al., Nucleic Acids Res. 31 (2003) e115; Gunderson et al., Nature Genetics 37 (2005) 549-554.

In a further preferred embodiment of the method of the invention, the physiological susceptibilities are selected from sensitivity to drug treatment, functional food, physical health schemes, identification of non-responsiveness to treatment by diet, medication or physical activity.

The term "sensitivity to drug treatment" in accordance with the invention relates to the response of an individual person to a drug that targets a given metabolic pathway. This response varies depending on the person's individual genotype in the corresponding genetically determined metabotype. For instance, a drug that directs more lipids into the beta-oxidation pathway is expected to show a different effect on different individuals, depending on the individuals' genotype in the SCAD, MCAD and LCAD polymorphisms. Based on these findings, the development of personalised medication is possible. Accordingly, the term "non-responsiveness to treatment by medication" relates to a decreased or missing effect observed by treatment with medication, wherein this effect is based on individual key pathways of the lipid metabolism or the overall homeostasis of the lipid metabolism of a person, which can be determined based on the genotypes of the polymorphisms in key lipid enzymes and transporters as identified in this invention. The effect of a medication that targets a change in the overall lipid homeostasis will depend on the strength with which the homeostasis is maintained, and may be inefficient in cases of specific combinations of the genetic variants in these key genes.

The term "sensitivity to functional food" in accordance with the invention relates to the ability or disability of a person to metabolize a given nutritional ingredient, wherein this sensitivity is dependant on the genotype in one or more key lipid enzymes and transporters as identified in this invention. As an example, this has been shown to be the case in the association between breastfeeding and IQ (Caspi et al., 2007). In the context of providing functional food, these findings allow the development of personalised nutrition, for example by providing a food specifically enriched in certain lipids, with the objective to increase a person's intellectual capacities. Accordingly, the term "non-responsiveness to treatment by diet" relates to the effect that a person may not respond as expected to a given diet due to a modified homeostasis of the lipid metabolism.

The term "sensitivity to physical health schemes" in accordance with the invention relates to the effect that a person may not or only slowly respond to weight reduction schemes by intense physical activity. For instance, this would be expected for carriers of the minor alleles of SCAD and MCAD, which would be expected to show a deficiency in directing fat into the beta-oxidation pathway by physical activity. The term "non-responsiveness to physical activity" relates, accordingly, to the case where a person does not respond at all to weight reduction schemes by intense physical activity.

The method of the present invention may furthermore be used for preclinical and clinical drug testing and toxicology studies. It is known that in such studies not all examined individuals respond equally to a given drug gavages. Based on the present study, it is expected that in specific cases such differential outcomes will be largely controlled by the individuals' genotype in their genetically determined metabotypes, in particular in situations where lipid metabolism-targeting drugs are used. By discriminating the individuals in the drug test by their genotypes, it is possible to identify groups that respond to the drug from those who do not, or who show toxic side effects. Moreover, this method may also be used for personal genome consulting, such as for example currently offered by companies such as 23andMe as well as in the promotion of functional foods that are adapted to personal genomes. It can also be used for genetic risk or ability assessment. For instance, it can be expected that individuals that are homozygous for the major alleles of both, the SCAD and the MCAD polymorphisms have a higher chance to become a high-performance sportive when trained accordingly, or that individuals that are homozygous for the minor alleles of these two polymorphisms present a higher risk of physical failure when they are put under starvation stress in combination with strong physical activity. In a further preferred embodiment of the method of the invention, the physiological susceptibilities results in a disease or condition selected from hyperactivity, a potential benefit from a specific nutrition (e.g. breast feeding and IQ) but also type 2 diabetes, metabolic syndrome, coronary artery disease, Crohn's disease, rheumatoid arthritis, border line syndrome and increased levels of cholesterol and triglycerides.

All of the diseases described herein are well known to the skilled person and are defined in accordance with the prior art and the common general knowledge of the skilled person.

The term "hyperactivity" relates to a physical state in which a person is abnormally and easily excitable or exuberant. Strong emotional reactions, impulsive behavior, and sometimes a short span of attention are also typical for a hyperactive person. Some individuals may show these characteristics naturally, as personality differs from person to person. Nonetheless, when hyperactivity starts to become a problem for the person or others, it may be classified as a medical disorder.

The term "type 2 diabetes" as used herein refers to a disease in which the body does not properly control the amount of sugar in the blood. As a result, the level of sugar in the blood is too high. This disease occurs when the body does not produce enough insulin or does not use it properly. Type II diabetes correlates with obesity and insulin resistance, and has its onset in the adult age. It is characterized by an inability of the islets of Langerhans to secrete sufficient insulin to compensate for the body's increased demand for insulin.

The term "metabolic syndrome" relates to a combination of medical disorders that increase the risk of developing cardiovascular disease and diabetes. It affects a great number of people, and prevalence increases with age. Metabolic syndrome is also known as metabolic syndrome X, syndrome X, insulin resistance syndrome or Reaven's syndrome. Symptoms and features are: Fasting hyperglycemia (diabetes type 2 or impaired fasting glucose, impaired glucose tolerance, or insulin resistance); hypertension; central obesity (also known as visceral, male-pattern or apple-shaped adiposity; overweight with fat deposits mainly around the waist); decreased HDL cholesterol or elevated triglycerides. Associated diseases and signs are: elevated uric acid levels, fatty liver (especially in concurrent obesity), progressing to non-alcoholic fatty liver disease, polycystic ovarian syndrome, hemochromatosis (iron overload); and acanthosis nigricans (a skin condition featuring dark patches). Different organizations dealing with diabetes have elaborated different definitions for metabolic syndrome. The World Health Organization (WHO) criteria (1999) require presence of diabetes type 2, impaired glucose tolerance, impaired fasting glucose or insulin resistance, and two of the following: blood pressure: ≥ 140/90 mmHg, dyslipidaemia: triglycerides (TG): ≥ 1.695 mmol/L and high-density lipoprotein cholesterol (HDL-C) ≤ 0.9 mmol/L (male), ≤ 1.0 mmol/L (female), central obesity: waist:hip ratio > 0.90 (male); > 0.85 (female), and/or body mass index > 30 kg/m², microalbuminuria: urinary albumin excretion ratio ≥ 20 mg/min or albumin:creatinine ratio ≥ 30 mg/g.

The term "coronary artery disease" (CAD) relates to a disease that occurs when part of the smooth, elastic lining inside a coronary artery develops atherosclerosis. With atherosclerosis, the artery's lining becomes hardened, stiffened, and swollen with e.g. calcium deposits, fatty deposits, and abnormal inflammatory cells to form a plaque. These plaques start limiting the blood flow to the heart muscle. Coronary artery disease is the end result of this accumulation of plaques within the walls of the arteries that supply the myocardium with oxygen and nutrients. CAD is the most common cause of sudden death, and is also the most common reason for death of men and women over 20 years of age.

The term "Crohn's disease" relates to an inflammatory disease of the digestive system which may affect any part of the gastrointestinal tract from mouth to anus. The disease is also known as granulomatous colitis and regional enteritis. Symptoms of Crohn's disease can vary significantly among afflicted individuals. The main gastrointestinal symptoms are abdominal pain, diarrhea (which may be visibly bloody), vomiting, or weight loss. Crohn's disease can also cause complications outside of the gastrointestinal tract such as skin rashes, arthritis, and inflammation of the eye. The precise cause of Crohn's disease is not known. The disease occurs when the immune system attacks the gastrointestinal tract. This autoimmune activity produces inflammation in the gastrointestinal tract, and therefore Crohn's disease is classified as an inflammatory bowel disease.

"Rheumatoid arthritis" in accordance with the present invention is an autoimmune disorder that causes the body's immune system to attack the bone joints (Muller B et al. 1998. Springer Semin Immunopathol. 20:181-96*).*

The term "border line syndrome" (BPD) relates to a psychiatric diagnosis that describes a prolonged disturbance of personality function characterized by depth and variability of moods. The disorder typically involves unusual levels of instability in mood; chaotic and unstable interpersonal relationships, self-image, identity, and behavior as well as a disturbance in the individual's sense of self. Attempted suicide and completed suicide are possible outcomes, especially without proper care and effective therapy. Onset of symptoms typically occurs during adolescence or young adulthood.

In a further preferred embodiment, the sample is blood, serum, plasma, fetal tissue, saliva, urine, mucosal tissue, mucus, vaginal tissue, fetal tissue obtained from the vagina, skin, hair or hair follicle.

The invention furthermore relates to a kit for determining a predisposition of a subject for physiological susceptibilities that result from alterations in lipid metabolism comprising a set of probes, wherein the set comprises at least two probes selected from the group consisting of a) a probe specifically binding to rs2014355; b) a probe specifically binding to rs11161510; c) a probe specifically binding to rs2286963; d) a probe specifically binding to rs174548; e) a probe specifically binding to rs9393903; f) a probe specifically binding to rs168622; g) a probe specifically binding to rs541503; h) a probe specifically binding to rs2046813; i) a probe specifically binding to rs272889; and j) at least one probe specifically binding to a genetic polymorphism that is in linkage disequilibrium with a genetic polymorphism selected from the group consisting of rs2014355; rs11161510; rs2286963; rs174548; rs9393903; rs168622; rs541503; rs2046813 and rs272889.

The term "probe" in accordance with the invention relates to a sensor molecule that may be used to detect the presence or absence of a target molecule. According to the present invention, such probes include, but are not limited to, nucleic acid molecules, antibodies or sequence-specific DNA-binding proteins.

Preferably, the probe is a nucleic acid molecule, preferably for use in molecular-biological hybridisation methods such as the sequence-specific detection of the genetic polymorphisms in accordance with the invention. The probes may be prepared with any of the methods known in the art, such as for example enzymatic production or according to conventional protocols of organic chemistry.

"Nucleic acid molecules", in accordance with the present invention, include naturally occurring nucleic acid molecules such as DNA or RNA. Further included are nucleic acid mimicking molecules known in the art such as synthetic or semisynthetic derivatives of DNA or RNA and mixed polymers, both sense and antisense strands. They may contain additional non-natural or derivatized nucleotide bases, as will be readily appreciated by those skilled in the art. In a preferred embodiment the polynucleotide or the nucleic acid molecule(s) is/are DNA. Such nucleic acid mimicking molecules or nucleic acid derivatives according to the invention include phosphorothioate nucleic acid, phosphoramidate nucleic acid, 2'-O-methoxyethyl ribonucleic acid, morpholino nucleic acid, hexitol nucleic acid (HNA) and locked nucleic acid (LNA) (see, for example, Braasch and Corey, Chemistry & Biology 8, 1-7 (2001)). LNA is an RNA derivative in which the ribose ring is constrained by a methylene linkage between the 2'-oxygen and the 4'-carbon.

For the purposes of the present invention, a peptide nucleic acid (PNA) is a polyamide type of DNA analog. The monomeric units for the corresponding derivatives of adenine, guanine, thymine and cytosine are available commercially (for example from Perceptive Biosystems). PNA is a synthetic DNA-mimic with an amide backbone in place of the sugar-phosphate backbone of DNA or RNA. As a consequence, certain components of DNA, such as phosphorus, phosphorus oxides, or deoxyribose derivatives, are not present in PNAs. As disclosed by Nielsen et al., Science 254:1497 (1991); and Egholm et al., Nature 365:666 (1993), PNAs bind specifically and tightly to complementary DNA strands and are not degraded by nucleases. Furthermore, they are stable under acidic conditions and resistant to proteases (Demidov et al. (1994), Biochem. Pharmacol., 48, 1310-1313). Their electrostatically neutral backbone increases the binding strength to complementary DNA as compared to the stability of the corresponding DNA-DNA duplex (Wittung et al. (1994), Nature 368, 561-563; Ray and Norden (2000), Faseb J., 14, 1041-1060). In fact, PNA binds more strongly to DNA than DNA itself does. This is probably because there is no electrostatic repulsion between the two strands, and also the polyamide backbone is more flexible. Because of this, PNA/DNA duplexes bind under a wider range of stringency conditions than DNA/DNA duplexes, making it easier to perform multiplex hybridization. Smaller probes can be used than with DNA due to the strong binding. In addition, it is more likely that single base mismatches can be determined with PNA/DNA hybridization because a single mismatch in a PNA/DNA 15-mer lowers the melting point (Tₘ) by 8°-20° C, vs. 4°-16° C for the DNA/DNA 15-mer duplex. Thereby discrimination between perfect matches and mismatches is improved. For its uncharged nature, PNA also permits the hybridisation of DNA samples at low salt or no-salt conditions, since no inter-strand repulsion as between two negatively charged DNA strands needs to be counteracted. As a consequence, the target DNA has fewer secondary structures under hybridisation conditions and is more accessible to probe molecules.
The term "derivatives" in conjunction with the above described PNAs and PNA chimera relates to molecules wherein these molecules comprise one or more further groups or substituents different from PNA and DNA. All groups or substituents known in the art and used for the synthesis of these molecules, such as protection groups, and/or for applications involving these molecules, such as labels and (cleavable) linkers are envisaged.

Said nucleic acid molecule probes are at least 10, preferably at least 15 such as at least 25 nucleotides long, if used for example as primers. Probes of the present invention to be used as a probe in Southern or Northern blot preferably comprise at least 50, more preferably at least 100, more preferably at least 200, and most preferably at least 500 nucleotides in length. Said probes may also be useful in primer extension protocols respectively. It is required that the probe is uniquely fitting to (hybridizing to/complementary to 100%) the sequences of the genetic polymorphism to be investigated in accordance with the present invention, but not to un-related sequences, so as to allow the detection of single mismatches.

Antibodies, in accordance with the present invention, have been described above.

The term "sequence-specific DNA-binding proteins" as used herein has also been described above.

The terms "specifically binds/binding to"" in accordance with the present invention relates to the sequence-specific binding of a probe to the respective genetic polymorphism, without cross-reactivity with any nucleic acid molecule sample that does not contain said genetic polymorphism. For example, in the case where the probe is a nucleic acid molecule, such specific binding maybe achieved by employing stringent hybridisation conditions as described above. The term "a probe specifically binding to ", as used in accordance with the invention, encompasses probes that are specific for either the major allele of the respective genetic polymorphism or for the minor allele. Thus, in a preferred embodiment, this term relates to a plurality of probes for both the major and the minor allele, that allow the skilled person to determine the genotype of a subject, i.e. whether the subject is heterozygous or homozygous for one of the alleles, with respect to the investigated genetic polymorphism.

The kit of the present invention allows the convenient and easy determination of the genotypic make-up of a subject. Based on the identified genotype and associated variations of the metabotype, as identified in accordance with the present invention, it is now possible to determine, using the kit of the invention, what the metabolic capacities of the individual subject are.

An envisaged application of the kit of the present invention is the provision of a kit-based device that will support a customer in making his daily choices of nutritional supplies in the super market, based on his individual genetic background and personal parameters, such as body mass index, physical fitness, health state, age, and gender. Such a device would be designed to run in real-time, for example on a mobile phone, by a customer while he visits a supermarket. This method could be realized in the following manner: in a first publicitary action, customers would be invited to donate a DNA sample (ideally sputum). The customer's DNA sample would then be analysed using the kit in accordance with the invention. The food company that sponsors this activity would then provide a web-service (ideally accessible online in the supermarket via a customized mobile phone application). This web service would provide the customer with the option to enter additional personal parameters that describe his health status, such as height, weight, age, gender, present physical activity, and potential health problems. The customer would enter the products or product types he intends to acquire. The web application would then combine the information of the individuals metabolic capacities, based on the genetic variants described in this invention, with the person's lifestyle parameters and the products he wishes to buy. Based on this information, the customer would obtain a suggestion of an individualized product choise that would be optimally adapted to his body's requirements. The functional dependence between the genetic variants, the lifestyle parameters, and effect of the proposed product choices on the outcome of the individual's health will be determined in a prior study, where the kit would also be used. Compliance could encouraged by proposing price reductions if the individualized product advice is followed.

A similar method could be followed for personalized medication. In this method a prior study would be performed on a patient cohort where the response to certain drugs as a function of metabolic capacities, characterized by the gene variants described in this invention, would be recorded. Then doctors would use this knowledge to subscribe personally adapted variants of certain drugs, that would be most efficient given the individual's metabolic capacities. Such studies could be conducted by companies that are independant from the drug producing pharma companies, and benefit could be drawn from selling access tot he knowledge bases that would be created in studies that use the set of gene variants of this invention (e.g. using the kit). Financial support could also be sought from public health insurance companies that have an interest in providing the most efficient treatment at the best price to their clients. One example could be the selection of lipid lowering drugs, such as statins, which have been shown to be beneficial even if given to a healthy person, by lowering her LDL cholesterol levels and thereby her risk of developping coronary artery disease.

The various components of the kit may be packaged in one or more containers such as one or more vials. The vials may, in addition to the components, comprise preservatives or buffers for storage.

In a preferred embodiment of the kit of the invention, the set of probes comprises probes for identifying the genotype of at least one genetic polymorphisms for each of the pathways identified in accordance with the present invention. Thus, probes are provided for the determination of the genotype of at least one of a) rs2014355 (SCAD), rs1116151 (MCAD) or rs2286963 (LCAD) for investigating the beta-oxidation of fatty acids; b) rs174548 (FADS1) or rs9393903 (ELOVL2) for investigating the metabolism of very long chain PUFAs; and c) rs168622 (SPTLC3) or rs541503 (PHGDH) for investigating the metabolism of sphingolipids.

In another preferred embodiment of the kit of the invention, the set of probes comprises at least the probes as defined in (a) to (i).

In a preferred embodiment of the kit of the invention, the set of probes is provided on a solid phase support.

As described above, the skilled person understands the term solid phase support. Non-limiting examples of a solid phase support include beads, chips and membranes.

In a preferred embodiment said solid phase support is a DNA chip. As described above, methods for the production of DNA chips as well as the use of DNA chip technology are well known in the art and described e.g. in Lohara et al., Nucleic Acids Res. 30 (2002) e87; Flavell et al., Nucleic Acids Res. 31 (2003) e115; Gunderson et al., Nature Genetics 37 (2005) 549-554.

The figures show:

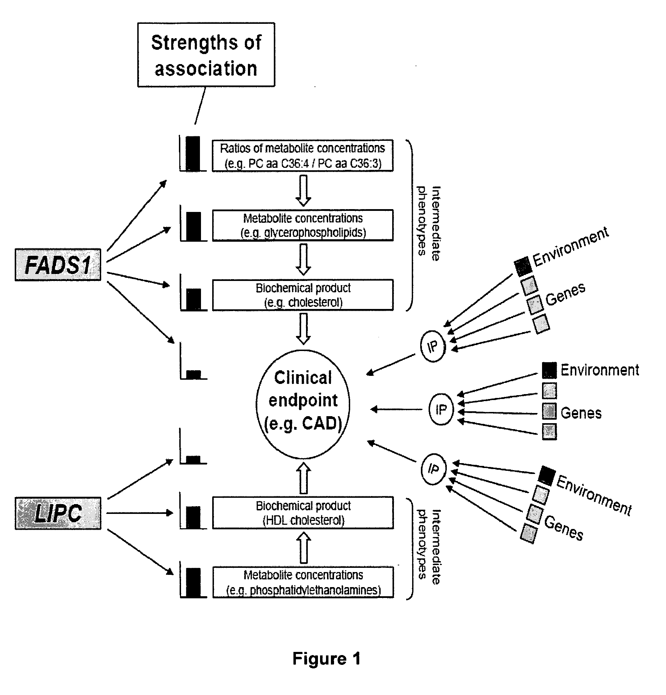

**Figure 1**: Schematic illustration of the role of intermediate phenotypes (IPs), such as metabolic traits, demonstrated at the examples of two genes that code for major enzymes of the long-chain fatty acid metabolism *(FADS1* and *LIPC*). We show that new information on the functional basis of the observed associations can be inferred from the biochemical properties of the affected metabolites. Moreover, both genes were previously reported to be associated with common clinical phenotypes, *FADS1* in an extent which would not attract immediate attention for follow-up in a genome-wide context. Since several genes and pathways are involved in the development of a clinical endpoint, the IP focuses on one pathway (e.g. cholesterol or a given metabotype) which is already known to be involved in the clinical endpoint (e.g. coronary artery disease (CAD)). It is much easier to identify the genes which are associated with the IP since the associations of genetic variation with the IP is much stronger than with the clinical endpoint. Environmental factors interact at different levels with the IPs and thereby add to the variability in the system. The closer the IP is related to the genetic polymorphism, the stronger the association is expected to be. In our case the association reflects enzymatic activity of *FADS1* and *LIPC* which results in very strong effect sizes of the genetically determined metabotype.

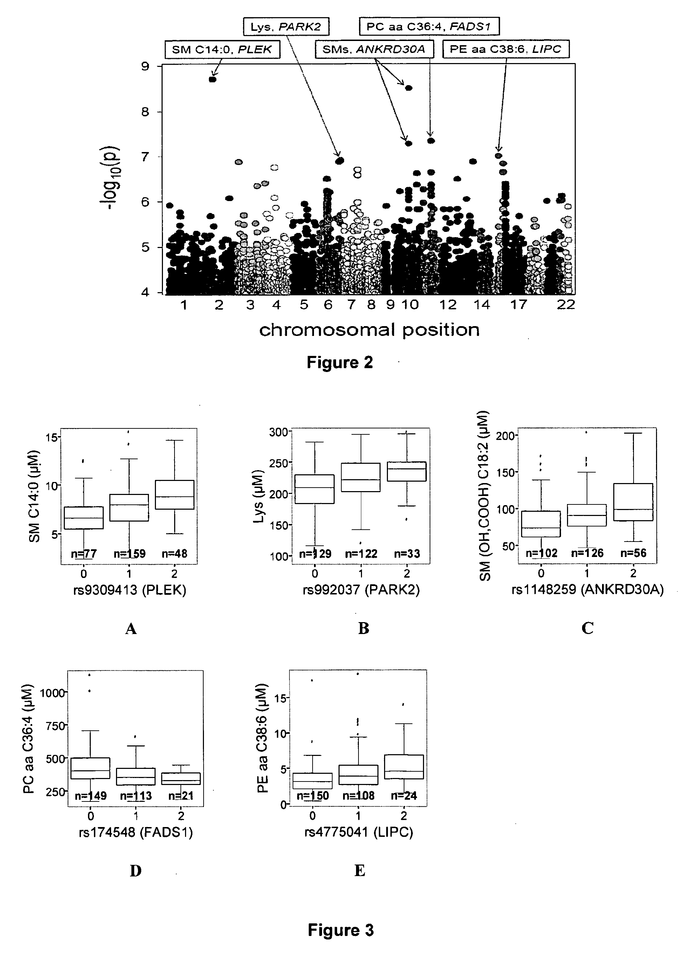

**Figure 2**: P-values of association assuming an additive genetic model, superposing the results obtained from all genome-wide tested metabolic traits. Chromosomal location is indicated by different colors on the x-axis, negative logarithmic p-values are reported on the y-axis. The top ranking SNPs together with the closest gene and the most significant associating metabolite(s) are indicated. A complete list of all associations with p<10⁻⁶ is provided in Table 5, together with significant associations from previous GWA studies with medical phenotypes. Metabolite abbreviations are explained in the material and methods section and a full list of all measured metabolites is provided as supplementary data.
**Figure 3**: Boxplots of the metabolite concentrations of five top ranking associations as a function of genotype. They show the differentiation of the population that is induced by these genetically determined metabotypes (0 = major allele homozygote, 1 = heterozygote, 2 = minor allele homozygote). Boxes extend from 1^{st} quartile (Q₁) to 3^{rd} quartile (Q₃); median is indicated as a horizontal line; whiskers are drawn to the observation that is closest to, but not more than, a distance of 1.5(Q₃-Q₁) from the end of the box. Observations that are more distant than this are shown individually on the plot. The number of individuals in each group is given below the boxes. P-values for these associations are given in Table 3.
**Figure 4**: Boxplots of the strongest associations of metabolite concentration ratios with polymorphisms in the FADS1 (a; p=2.4x10⁻²²), SCAD (b; p=9.3x10⁻¹⁷), and MCAD (c; p=7.6x10⁻¹⁷) genes (see legend to Fig. 3 for details). The metabolic efficiencies of the reactions that are catalyzed by these three enzymes differ considerably between individuals of different genotype.
**Figure 5**: P-values of association assuming an additive genetic model, superposing the results obtained from all genome-wide tested metabolic concentration ratios for the replication study, limited to SNPs with a minor allele frequency greater than 20%, that do not violate the Hardi-Weinberg equilibrium (p>10⁻⁶), and that have a call rate larger than 95%. All identified lipid metabolism-related proteins with a p-value of association smaller than 10⁻¹⁰ are annotated..
**Figure 6**: Boxplots of the strongest associations of metabolite concentration ratios with polymorphisms in the SCAD, MCAD, LCAD, FADS1, ELOVL2, SPTLC3, PHGDH, ACSL1, and OCTN1 genes, as identified in Figure 5, for the replication study with 1048 individuals are shown.

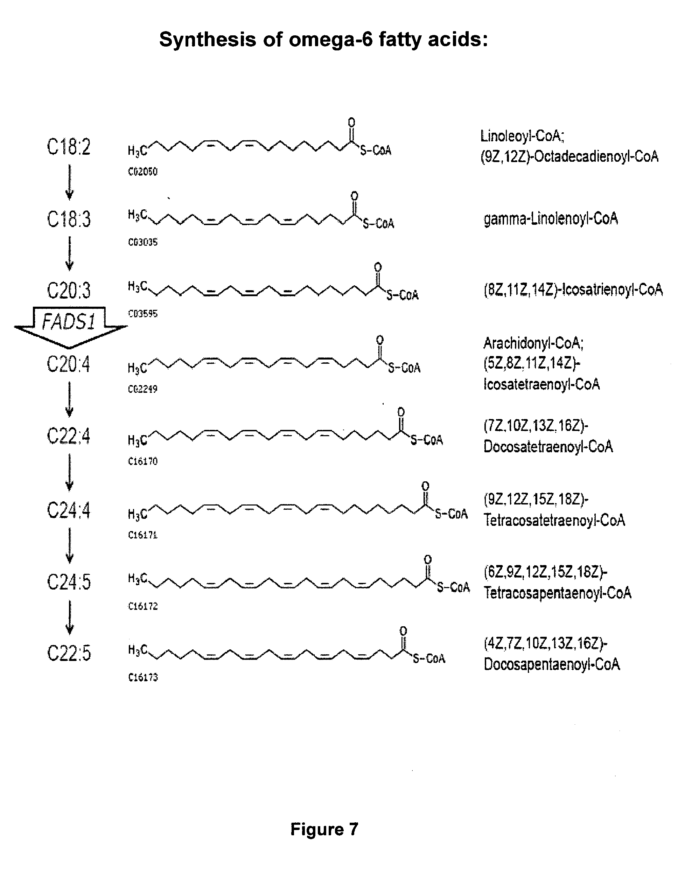

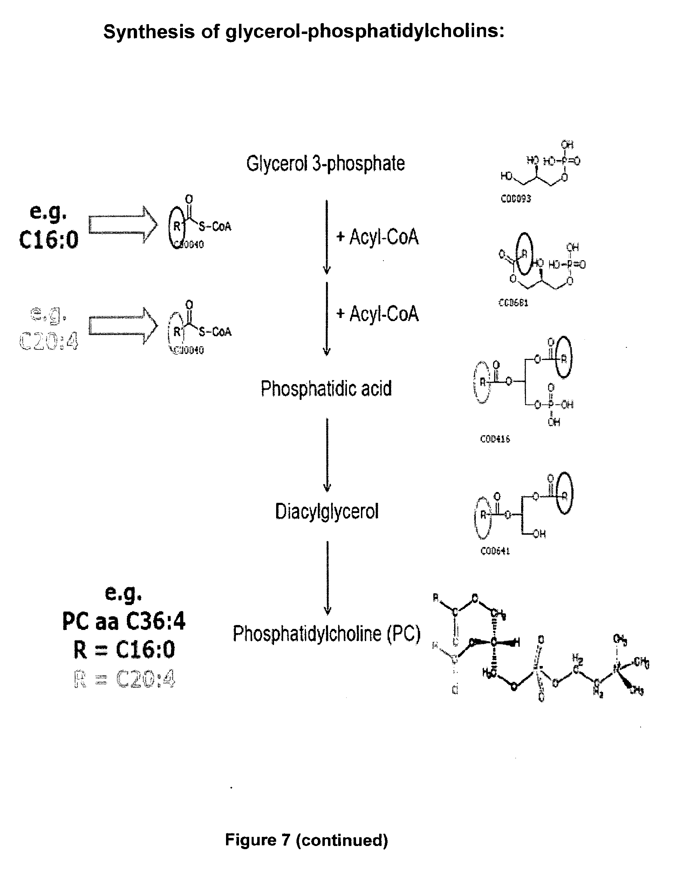

**Figure 7**: Schematic view of the role of *FADS1* in the synthesis of phosphatidylcholine. Long-chain poly-unsaturated fatty acids have to be produced from the essential fatty acid linoleic acids (C18:2) in the omega-6 fatty acid synthesis pathway (top figure) and from alpha-linolenic acid (C18:3) in the omega-3 fatty acid synthesis pathway (not shown). Un- and monosaturated fatty acids with chain lengths of up to 18 carbons, i.e. palmic acid (C16:0), stearic acid (C18:0) and oleic acid (C18:1) can be synthesized de novo in the human body. In the Kennedy pathway, glycerol-phosphatidylcholins (PC) with different fatty acid side chains are then produced from two fatty acid moieties (bottom figure). These are linked to a glycerol 3-phosphate, followed by a dephosphorylation step and the addition of a phosphocholin moiety. Figures and pathways shown here were adapted from the KEGG database at http://www.genome.jp/kegg/ (Kanehisa et al. 2006).

The examples illustrate the invention.

### Example 1: MATERIAL AND METHODS

**Study population.** This study is based on a previously reported genotyping effort (Döring et al. 2008; Heid et al. in press) whereof we report the essentials here. We recruited the study population for the genome-wide association study from the KORA S3 survey that is a population-based sample from the general population. The dataset comprises individuals aged 25-74 years resident in the region of Augsburg, Southern Germany, examined in 1994-1995. The standardized examinations applied have been described in detail elsewhere (Wichmann et al. 2005 and references therein). We selected 1,644 subjects, who participated in a follow-up examination of S3 (F3 500K), comprising individuals who, at that time, were aged 35-79 years. With regard to possible effects from population stratification it should be noted that previous work with the KORA F3 500K dataset excluded population stratification as the origin of an observed association with uric acid on the basis of comparison with two other studies (Döring et al. 2008). Moreover, possible population stratification in KORA F3 500K was also excluded based on an EIGENSOFT analysis performed in an earlier independent report. Also, recent experimental assessment has found little population stratification to exist within and across Germany.

**Genotyping.** Genotyping for KORA F3 500K was done using the Affymetrix 500K Array Set, consisting of two chips *(Sty* / and *Nsp* /). Hybridization of genomic DNA was done in accordance with the manufacturer's standard recommendations. Genotypes were determined using the BRLMM clustering algorithm (hftp://www.affymetrix.com/support/technical/whitepapers/brlmm whitepaper.pdf). The genotypes were determined in batches of at least 400 chips. For quality control purposes, we applied a positive and a negative control DNA every 48 samples. The overall genotyping efficiency of the GWA was 98.26 %. Before statistical analysis, we performed filtering of both conspicuous chips and SNPs based on quality measures to ensure robustness of association analysis. On chip level only subjects with overall genotyping efficiencies of at least 93% for both chips and at most one discordant call for 50 SNPs situated on both chips were included. In addition the called gender has to agree with the gender in the KORA study database. On SNP level from a total of 500,568 SNPs, we excluded for the purpose of this study all SNPs on chromosome X leaving 490,032 autosomal SNPs for the GWA screening step. From these 187,454 SNPs (38.25%) passed all subsequent filter criteria, and were selected for the association analyses presented in this paper. Criteria leading to exclusion were genotyping efficiency < 95% (N = 49,325) and genotype frequency of the minor genotype < 5% (N = 252,405). An exact Fisher test was used to detect deviations from Hardy Weinberg Equilibrium, and we excluded all SNPs with P-values below 10⁻⁶ (N = 848 after passing the other criteria).

**Sampling.** From the 1644 participants genotyped in the KORA F3 500K study population, 284 males (55-79 years) were selected at random for metabolic characterization. Blood samples for metabolic analysis were collected during 2006. To avoid variation due to circadian rhythm, blood was drawn in the morning between 8 and 10 am after a period of overnight fasting. Material was immediately horizontal shaken (10 min), followed by 40 min resting at 4°C to obtain complete coagulation. The material was then centrifuged (2000g; 4°C). Serum was aliquoted and kept for 2-4 hours at 4°C, after which it was deep frozen to -80°C until sampling.

**Metabolite measurements.** Targeted metabolite profiling by electrospray ionization (ESI) tandem mass spectrometry (MS/MS) was performed on a fee-for-service basis on a quantitative metabolomics platform at *Biocrates Life Sciences* AG, Austria. The company had no access to genotype or phenotype information that would have permitted any data pre-filtering other than objective quality control for measurement errors based on internal controls and duplicates. All metabolomics data was used as received from Biocrates. We did not apply any data correction, nor were any data points removed. The experimental metabolomics measurement technique is described in detail by patent US 2007/0004044 (accessible online at hftp://www.freepatentsonline.com/20070004044.html). Briefly, a targeted profiling scheme is used to quantitatively screen for known small molecule metabolites using multiple reaction monitoring, neutral loss and precursor ion scans. Quantification of the metabolites of the biological sample is achieved by reference to appropriate internal standards. The method has been proven to be in conformance with 21CFR (Code of Federal Regulations) Part 11, which implies proof of reproducibility within a given error range. It has been applied in different academic and industrial applications (Altmaier et al. 2008). Concentrations of all analyzed metabolites are reported in µM (except for prostaglandin concentrations which are reported in nM units).

**Metabolite panel.** In total, 363 different metabolites were detected (shown in Table 4). The metabolomics dataset contains 18 amino acids, nine reducing mono-, di- and oligosaccharides (abbreviated as Hn for n-hexose, dH for desoxyhexose, UA for uronic acid, HNAc for N-acetylglucosamine, P for Pentose, NANA for N-acetylneuraminic-acid), seven biogenic amines, five prostaglandins, arachidonic acid (AA), docosahexaenoic acid (DHA), free carnitine (C0), 28 acylcarnitines (C*x:y*), hydroxylacylcarnitines (C(OH)*x:y*), and dicarboxylacylcarnitines (C*x:y*-DC), 85 ceramides (Cer), glucosylceramides (GlcCer), different sphingomyelins (SM*x:y*) and sphingomyelin-derivatives, such as N- hydroxyldicarboacyloylsphingosyl-phosphocholine (SM(OH,COOH)*x:y*) and N- hydroxylacyloylsphingosyl-phosphocholine (SM (OH)*x:y*). In addition, 208 phospholipids were detected, including different glycero-phosphatidic acids (PA), glycero-phosphatidylcholines (PC), glycerophosphatidylethanolamines (PE), phosphatidylglycerols (PG), glycero-phosphatidylinositols (PI), glycero-phosphatidylinositol-bisphosphates (PIP2), and glycero-phosphatidylserines (PS). Glycerophospholipids are further differentiated with respect to the presence of ester (a) and ether (e) bonds in the glycerol moiety, where two letters (aa=diacyl, ae=acyl-alkyl, ee=dialkyl) denote that two glycerol positions are bound to a fatty acid residue, while a single letter (a=acyl or e=alkyl) indicates the presence of a single fatty acid residue. Lipid side chain composition is abbreviated as Cx:y, where x denotes the number of carbons in the side chain and y the number of double bonds. E.g. "PC ea C33:1" denotes a plasmalogen/plasmenogen phosphatidylcholine with 33 carbons in the two fatty acid side chains and a single double bond in one of them. The precise position of the double bonds and the distribution of the carbon atoms in different fatty acid side chains cannot be determined with this technology. In some cases, the mapping of metabolite names to individual masses can be ambiguous. For example, stereo-chemical differences are not always discernible, neither are isobaric fragments. In such cases, possible alternative assignments are indicated.

**Statistical analysis.** In the statistical analysis only SNPs with a minor allele homozygote frequency of at least 5% were included in order to account for the relatively small sample size of the study. The corresponding smallest minor allele frequency (MAF) in the analyzed dataset is 18.2%. In a first full genome-wide screen, metabolites with less than 5% missing values were used (201 metabolite variables). Additive genetic models assuming a trend per copy of the minor allele were used to specify the dependency of metabolites on genotype categories in the genome wide association study. No further adjustment was performed. The linear regression algorithm implemented in the statistical analysis system R (http://www.r-proiect.org/) was used in the genome wide association study and SPSS for Windows (Version 15.0, Chicago: SPSS Inc.) was used for statistical analysis on a case-by-case level. It should be noted that the calculation of p values is based on asymptotic assumptions, which do not apply down to extremely low levels. Such p-values should thus be interpreted merely as indicators for the strength of an association, but not as absolute probabilities. A conservative estimate of a genome-wide significance level (using the Bonferroni correction) based on a nominal level of 0.05 is 1.33x10⁻⁹ (0.05 / (201*187,454)). However, such a small p-value of an association would only be required to confirm an association between a SNP and a single metabolite concentration if all SNPs and metabolites were acting independently. In the case of a GWA study with metabolomics, evidence from multiple metabolic traits can be combined into a multi-factorial "metabolic story", where changes in metabolite concentrations are interpreted in the context of their position on the metabolic pathways. To document the complete story all SNPs that associate with at least one metabolic trait, associations with a p-value smaller than 10⁻⁴ are retained for further analysis (2927 SNPs). These have been selected from a set of 187,454 SNPs. Moreover, the metabolic measures are not independent, and therefore if by chance one trait associates with an SNP its correlate would also be expected to associate with that SNP. For the top ranking associations we then carried out a linear regression between the associating SNP (additive genetic model), using all available (max. 363) metabolite concentrations as quantitative traits. In addition, we computed all possible pairs of metabolite concentration ratios for those cases and used those ratios as quantitative traits in a subsequent test. A strong reduction in p-value indicates that two metabolites may be linked by a metabolic pathway that is modified by the SNP. A conservative estimation of the genome-wide significance level (Bonferroni correction) when testing all metabolite pairs, based on a nominal level of 0.05, is 6.6x10⁻¹² (1.33x10⁻⁹/201).

**GWA data from other studies.** Genome wide association data (p-values) from three recent GWA studies was downloaded on 21 February 2008 from http://www.broad.mit.edu/diabetes/scandinavs/metatraits.html (Broad Institute (Kathiresan et al. 2008)) and http://www.sph.umich.edu/csg/abecasis/public/lipids/ (University of Michigan (Willer et al. 2008)) and on 14 March 2008 from http://www.wtccc.org.uk/info/summary stats.shtml (Wellcome Trust Case Control Consortium (2007)). All p-values of association of these three GWA studies were combined with our dataset. In the WTCCC study several methods to compute p-values of association were used. Here we only use p-values using the additive frequentist model on the base population (controls and suitable cases merged as described in (WTCCC 2007)). Data points from that study that were flagged as having bad clustering parameters in the genotype calling were excluded.

### Example 2: Genome-wide association study with metabolic traits

A quantitative metabolomics platform based on electrospray ionization (ESI) tandem mass spectrometry (MS/MS) was chosen to determine the fasting serum concentrations of up to 363 endogenous metabolites, including nine sugar molecules, seven biogenic amines, seven prostaglandins, 29 acylcarnitines, 18 amino acids, 85 sphingolipids, and 208 glycerophospholipids (metabolite naming conventions are defined in the Material and Methods section above and a full list of all measured metabolites is provided in Table 3). Data for 201 of these metabolites was obtained for more than 95% of the samples. We conducted a genome-wide association study with these metabolic traits in a group of 284 randomly selected population-based male individuals between 55 and 79 years from the KORA F3 study (Wichmann et al. 2005). Single nucleotide polymorphisms were determined previously on a genome-wide scale for this population using the Affymetrix GeneChip Human Mapping 500K Array Set (Döring et al. 2008; Heid et al. in press). To avoid false positive effects from associations based on small numbers, we limited our analysis to SNPs in which at least 5% of the population is homozygous for the minor allele. The corresponding minor allele frequencies are > 18.2%. The resulting p-values of association for all metabolites when using an additive genetic model are presented in Figure 2. After correction for testing multiple loci and multiple metabolic traits, we estimated a conservative genome-wide level of significance of at least 1.33x10⁻⁹. None of the associations that we found attained that level when considering isolated metabolic traits. However, the best SNPs rs9309413 (p=1.95x10⁻⁹; Table 9) 21 kb upstream of the *PLEK* gene and rs1148259 (p=3.04x10⁻⁹; Table 10) in the 3'UTR of *ANKRD30A* were only slightly above genome-wide significance. This is notable because, in contrast to most previous GWA studies, in which association with few and mostly independent phenotypes was tested, a GWA study with metabolomics tests multiple and functionally related outcomes. Moreover, we will show in the following that signals of genome-wide significant levels (p-values between 10⁻¹⁶ and 10⁻²¹)can be attained when ratios between metabolite concentrations are used, and that some of our associations can also be considered as true positives on biological grounds.

In a follow-up study (called "replication study" hereafter) we measured a panel of 163 metabolites, using the *Biocrates AbsolutelDQ* kit, in a population comprising a total of 1048 individuals, including male and female individuals aged between 35 and 75. Genotyping was performed on an *Affymetrix -* Genome-Wide Human SNP Array 6.0. Dataanalysis was performed as in the initial study. The identified SNPs are reported in Figures 5 and 6.

### Example 3: A prototype of a genetically determined metabotype: FADS1

We started our analysis by considering polymorphisms in functionally well characterized enzymes that are among the top ranking association signals in our GWA study (Tables 4 and 5). SNP rs174548, one of several SNPs that lie in a linkage disequilibrium block containing the *FADS1* gene was strongly associated (up to p=4.52x10⁻⁸) with a number of glycerophospholipid concentrations (Figure 3 and Table 6). This SNP explains up to 10% of the observed variance of certain glycerophospholipids. The *FADS1* gene codes for the *fatty acid delta-5 desaturase,* a key enzyme in the metabolism of long-chain polyunsaturated omega-3 and omega-6 fatty acids (a schematic illustration of this pathway is presented in Figure 7). The minor allele variant of this SNP (MAF 27.5%) results in a reduced efficiency of the fatty acid delta-5 desaturase reaction, a fact that can be inferred from the following observations: the concentrations of numerous phosphatidylcholines (PC aa C34:4, PC aa C36:4, PC aa C36:5, PC aa C38:4, PC aa C38:5, PC aa C38:6, PC aa C40:4, PC aa C40:5; metabolite abbreviations are explained in the material and methods section), plasmalogen/plasmenogen phosphatidylcholines (PC ae C36:4, PC ae C38:4, PC ae C38:5, PC ae C38:6, PC ae C40:5), and the phosphatidylinositol PI aa C38:4 with four and more double bonds in their polyunsaturated fatty acid (PUFA) side chains are lowest in individuals that carry the minor allele of rs174548. In particular, the concentrations of the direct product of *FADS1,* arachidonic acid as well as those of its lyso-phosphatidylcholine derivative (PC a C20:4) are found to be significantly reduced with increasing copy number of the minor allele. On the other hand, concentrations of glycerophospholipids with three and less double bonds in their PUFA side chains show a positive association with the *FADS1* genotype. These metabolites include the phosphatidylcholines PC aa C34:2 and PC aa C36:2, the plasmalogen/plasmenogen phosphatidylcholines PC ae C34:2 and PC ae C36:2, the phosphatidylethanolamines PE aa C34:2 and PE aa C36:2, and the phosphatidylinositol PI aa C36:2. The negative association of the sphingomyelin concentrations (SM C22:2, SM C24:2, SM C28:4) can be interpreted as being a result of a changed homeostatis of phosphatidylcholins, since sphingomyelin can be produced from phosphatidylcholine by the action of the sphingomyelin synthase. Similarly, the negative association of the lyso-phosphatidylethanolamin PE a C10:0 can be considered a consequence of the overall changed balance in glycerophospholipid metabolism, since this metabolite can be produced from different phosphatidylethanolamines by abstraction of an arachidonic acid moiety. In summary, we can conclude that the direction of all those associations can be explained by a modification in the efficiency of the fatty acid delta-5 desaturase reaction.

### Example 4: Ratios of metabolite concentrations increase the power of association

Analyzing ratios of metabolite concentrations may strongly reduce the variation in the dataset when a pair of metabolites is closely connected to the direct substrates and products of a given enzymatic reaction. When a tested SNP impacts the efficiency of such a metabolic reaction, using concentration ratios leads to drastically decreased variance, and, consequentially, strongly decreased p-values of associations. Such a dependency not only provides rational evidence for a positive association, but also points to potentially affected metabolic pathways, as we demonstrate here for the example of the *FADS1* case. We find that by using metabolite concentration ratios, the p-value of the association with the polymorphism in the *FADS1* gene decreases by up to fourteen orders of magnitude (Table 7). Eicosatrienoyl-CoA (C20:3) and arachidonyl-CoA (C20:4) are the direct substrate and product of the delta-5 desaturase reaction, which is catalyzed by *FADS1.* Synthesis of these metabolites to a glycerol 3-phosphate, and further addition of a palmitoyl-moiety (C16:0), followed by a dephosphorylation step and the addition of a phosphocholin moiety, leads to the formation of the glycerol-phosphatidylcholins PC aa C36:3 and PC aa C36:4, respectively (for a schematic view of the phosphatidylcholine biosynthesis at the example of PC aa C36:4 see Fig. 7). PC aa C36:3 and PC aa C36:4 can thus be considered as modified substrates and products of the delta-5 desaturase reaction. If the catalytic activity (or the protein abundance) of *FADS1* is reduced by a polymorphism in its gene (or in a regulatory element), more eicosatrienoyl-CoA (C20:3) and less arachidonyl-CoA (C20:4) is available for the synthesis of those glycerophospholipids that contain these fatty acids. This translates for example into increased PC aa C36:3 concentrations and reduced PC aa C36:4 concentrations. Thus, the ratio between the concentrations of the product-substrate pairs of the delta-5 desaturase reaction, such as [PC aa C36:4]/[ PC aa C36:3] (Figure 4a), will be a strong indicator for the efficiency of the *FADS1* reaction. As reported in Table 7, glycerophospholipids with three double bonds do not associate with the *FADS1* polymorphism (p-values ranging from 0.92 to 0.077), whereas the corresponding glycerophospholipids with four double bonds generally display strong associations (most p-values ranging from 10⁻³ to 10⁻⁸). When considering the ratios between concentrations of matched metabolite pairs, the association with the polymorphism in the FADS1 gene increases by up to fourteen orders of magnitude (p-values below 10⁻²¹). This effect is observed not only for one, but for a number of different glycerophospholipid species (PC, PE, PI, incl. plasmalogen/plasmenogen phospholipids) which are thus very likely composed of an arachidonyl-moiety (C20:4) and either a palmitoyl- (C16:0) or a stearoyl-moiety (C18:0), respectively (except for lyso-phosphatidylcholin PC a C20:4, which is formed from a single arachinodyl-moiety). The strongest effect size is observed for phosphatidylcholine diacyl C36:4 (PC aa C36:4) to phosphatidylcholine diacyl C36:3 (PC aa C36:3) ratio (p=2.4x10⁻²²). These metabolites are major constituents of the cell membrane. Here, 28.6% of the total variance in the population can be explained by this SNP (Table 7 and Figure 4a). If the molecular function of *FADS1* had not been already known, the association between the SNP and the different glycerophospholipid concentrations per se would have allowed to deduce its enzymatic activity of inserting a fourth double bond into long-chain fatty acids.

### Example 5: Association with medical phenotypes

Having shown that this polymorphism in the *FADS1* gene strongly influences the serum glycerophospholipid homeostasis, we investigated the effect of this variation on biochemical variables related to medical outcomes. As glycerophospholipids play a major role in cholesterol metabolism, we hypothesized that the *FADS1* polymorphism should have a detectable effect on the corresponding serum parameters when looking at a sufficiently large population. This is indeed the case. Two recent GWA studies with up to 18,000 participants (Kathiresan et al. 2008; Willer et al. 2008) report p-values of association for SNP rs174548 with serum low-density lipoprotein (LDL) cholesterol, high-density lipoprotein (HDL) cholesterol and total cholesterol levels that range between 1.89x10⁻⁴ and 6.07x10⁻⁵ (Table 4). These associations have not been included into the list of potential candidates for replication in those studies, as their p-values taken alone were not sufficiently small in the context of a "classical" GWA study. Our association of SNP rs174548 with different glycerophospholipids can be viewed as an indirect replication of the association of *FADS1* with HDL, LDL and total cholesterol levels in an independent population. Furthermore, we can now hypothesize that the observed change in cholesterol levels induced by this SNP is functionally related to the availability of polyunsaturated long-chain fatty acids with four and more double bonds and its impact on the homeostasis of different glycerophospholipids. This case shows that a combination of a GWA study using metabolomic phenotypic traits with data from previous GWA studies can make it possible to identify promising new candidate SNPs associated to known phenotypes of medical relevance, and to gain new insight into the functional background of these associations.

### Example 6: A second genetically determined metabotype that associates with medical phenotype: LIPC

We therefore screened in a further step our strongest associations for overlap with associations in three recent large GWA studies, including serum lipid parameters well known to be involved in cardiovascular diseases as well as seven major common disease phenotypes (WTCCC 2007; Kathiresan et al. 2008; Willer et al. 2008). Following this strategy, we identified a series of SNPs in which the biochemical properties of the associated metabolites support the previously reported associations with their clinical outcomes (Tables 4 and 5). One example is SNP rs4775041, which is also in the list of our top ranking associations. This SNP is located in a linkage disequilibrium block containing the gene coding for *LIPC,* a key enzyme of the long-chain fatty acid metabolism. This polymorphism associates with the concentrations of numerous glycerophosphatidylcholines, glycerophosphatidylethanolamines and sphingomyelins (up to p=9.66x10⁻⁸; Figure 7 and Table 8). For instance, homozygotes carrying the minor allele have on average 70% higher concentrations of the phosphatidylethanolamine diacyl C38:6 (PE aa C38:6) than homozygotes for the major allele. The molecular function of *LIPC* is to break-down triglycerides to diacyl- and monoacylglycerols and fatty acids, which makes this association functionally plausible. In previous GWA studies this locus was reported to be associated with HDL cholesterol (p=2.80x10⁻⁹, 3.0x10⁻⁵, 2.0x10⁻³, and 7.0x10⁻³) and triglyceride levels (p=7.30x10⁻⁵) (Kathiresan et al. 2008; Wallace et al. 2008; Willer et al. 2008; Heid et al. in press).

These results thus not only prove the association of *LIPC* with HDL cholesterol and triglyceride levels in an independent population, but, similar to the FADS1 case, they provide new insights into the undenying biochemical mechanism of this association by identifying the involved lipid metabolites. Here we find phosphatidylethanolamines as the most strongly affected metabolites, prompting further research on their role in the cholesterol pathway. For instance, one may speculate that the substrate specificity of *LIPC* is affected by this genetic polymorphism. Interestingly, SNP rs4775041 also weakly associates with type 2 diabetes (p=0.061), bipolar disorder (p=0.048) and rheumatoid arthritis (p=0.059), and this in a third, independent population (WTCCC 2007). These associations are not significant on a genome-wide scale. However, the associations of this polymorphism with phospholipids reported here, as well as its associations with blood cholesterol levels in independent studies suggests that this genetic variant may indeed be causally related to these diseases, albeit further studies in larger populations will be needed to test this hypothesis. In any case, this example indicates how metabolic traits may serve as intermediate phenotypes to identify potential links between genetic variance and complex diseases (see Fig. 1).

### Example 7: PARK2 and PLEK

It is noteworthy that we could identify and validate two associations *(FADS1* and *LIPC)* with major genetically determined metabotypes (concentrations of metabolites and concentration ratios) among the five strongest associations in our GWA study despite the moderate number of participants in this study. We attribute this fact to the unexpectedly large effect sizes in combination with small variances of the genotype-metabotype associations. As it is evident that a number of the other top ranking candidate associations provide information relevant for causal genotype/phenotype associations, we report these results to serve as a resource for further research (Table 5). To give some illustrative examples, a polymorphism in the *PARK2* gene (rs992037; also among the five strongest associations) alters the concentrations of several amino acids. Some of these amino acids are directly connected to the urea cycle (Table 12). *PARK2* codes for parkin, a ubiquitin ligase for which a loss-of-function mutation has been reported to result in Parkinson's disease. When using ratios between metabolite concentrations we observed up to three orders of magnitude smaller p-values (Table 12). This suggests that this polymorphism impacts some metabolic pathway that involves glutamate on the one hand and a number of other amino acids (except lysine) on the other hand. Thus, the metabolic footprint of this association is that of an amino acid interconversion, which is supported by the functional role of *PARK2* as a ubiquitin ligase in the protein degradation pathway. Another example for a biologically plausible association is SNP rs9309413, which lies 21 kb upstream of *PLEK.* This SNP has the lowest p-value of association in this study (p=1.95x10⁻⁹). The *PLEK* gene codes for pleckstrin, a protein that has been proposed to facilitate protein/lipid interactions and to affect membrane structure. The polymorphism we report here impacts on a number of sphingomyelins, which are known to play a major role in membrane lipid structure (Table 7).

### Example 8: SCAD and MCAD

Prompted by the strong increase in the association signal in the FADS1 example by using metabolite concentration ratios, we tested the ratios of all possible metabolite pairs for association with any of the SNPs that have a minor allele frequency higher than 20%. We identified two new loci that are comparable to the FADS1 example in their strength of association and also in terms of the metabolic traits matching the genes' function. The first polymorphism is located in the gene coding for the short-chain acyl-Coenzyme A dehydrogenase (SCAD; e.g. intronic SNP rs2014355, minor allele frequency 25.1 %), located on chromosome 12, and the second lies in the gene coding for the medium-chain acyl-Coenzyme A dehydrogenase (MCAD; e.g. intronic SNP rs11161510, minor allele frequency 31.2%) on chromosome 1. Coincidentally, both genes code for enzymes that initiate the beta-oxidation of fatty acids, but they differ in the preference for their chain lengths. The metabolite pair that associates most strongly with rs2014355 of SCAD is the ratio between the short-chain acylcarnitines C3 and C4 (p=9.3x10⁻¹⁷, explained variance 21.8%, Fig. 4b) while the pair that associates most strongly with rs11161510 of MCAD is the ratio between the medium-chain acylcarnitines C12 and C8 (p=7.6x10⁻¹⁷, explained variance 21.9%, Fig. 4c). Fatty acids are bound to free carnitine for transport and beta-oxidation into the mitochondria. Similar to our argumentation in the FADS1 example, we can therefore consider the short-chain acylcarnitines as indirect substrates and products of SCAD and the medium-chain acylcarnitines as indirect substrates of MCAD, which matches the biochemical function of these enzymes. From the direction of the effect of these polymorphisms (higher concentrations of the longer chain fatty acids (=substrates) when compared to the smaller chain fatty acids (=products) implies a reduced dehydrogenase activity) we can further deduce that in both cases minor allele homozygotes have the lowest enzymatic turnover for these reactions.

### Tables

**Table 3: Metabolite panel used in this study**

| **Metabolite abbreviation (as used by Biocrates)** | **Metabolite class** | **Metabolite name** |
|---|---|---|
| C0 | Acylcarnitine | Carnitine (free carnitine) |
| C10 | Acylcarnitine | Acylcarnitine |
| C10:1 | Acylcarnitine | Acylcarnitine |
| C11 | Acylcarnitine | Acylcarnitine |
| C12 | Acylcarnitine | Dodecanoylcarnitine |
| C12:1 | Acylcarnitine | Dedecenoylcarnitine |
| C14 | Acylcarnitine | Acylcarnitine |
| C14:1 | Acylcarnitine | Tetradecenoylcarnitine |
| C14:2 | Acylcarnitine | Tetradecadienoylcarnitine |
| C16 | Acylcarnitine | Palmitoylcarnitine |
| C16:1 | Acylcarnitine | Acylcarnitine |
| C18 | Acylcarnitine | Stearoylcarnitine |
| C18:1 | Acylcarnitine | Oleoylcarnitine |
| C18:2 | Acylcarnitine | Linileylcarnitine |
| C18:2-OH | Acylcarnitine | Hydroxylacylcarnitine |
| C2 | Acylcarnitine | Acetylcarnitine |
| C3 | Acylcarnitine | Propionylcarnitine |
| C3-DC | Acylcarnitine | Malonylcarnitine |
| C3-DC-M | Acylcarnitine | Methylmalonylcarnitine |
| C4 | Acylcarnitine | Butyrylcarnitine |
| C4:1-DC | Acylcarnitine | Malyl-fumarylcarnitine |
| C5 | Acylcarnitine | Isovalerylcarnitine |
| C5:1-DC | Acylcarnitine | Acylcarnitine |
| C5-DC | Acylcarnitine | Dicarboxylacylcarnitines |
| C5-M-DC | Acylcarnitine | Methylglutarylcarnitine |
| C6 | Acylcarnitine | Hexanoylcarnitine |
| C8 | Acylcarnitine | Octanylcarnitine |
| C8:1 | Acylcarnitine | Octenoylcarnitine |
| C9 | Acylcarnitine | Acylcarnitine |
| ALA | Amino Acid | Alanine |
| ARG | Amino Acid | Arginine |
| ASP | Amino Acid | Aspartate |
| CIT | Amino Acid | Citrulline |
| GLU | Amino Acid | Glutamate |
| GLY | Amino Acid | Glycine |
| HIS | Amino Acid | Histidine |
| LYS | Amino Acid | Lysine |
| MET | Amino Acid | Methionine |
| ORN | Amino Acid | Ornithine |
| PHE | Amino Acid | Phenylalanine |
| PRO | Amino Acid | Proline |
| SER | Amino Acid | Serine |
| THR | Amino Acid | Threonine |
| TRP | Amino Acid | Tryptphan |
| TYR | Amino Acid | Tyrosine |
| VAL | Amino Acid | Valine |
| XLEU | Amino Acid | (Iso)Leucine |
| HNAc-H2-dH | Sugar | HNAc-H2-dH |
| HNAc-H4 | Sugar | HNAc-H4 |
| P | Sugar | Pentose |
| UA | Sugar | Uronic acid |
| DH | Sugar | Desoxyhexose |
| H1 | Sugar | Hexose |
| H2 | Sugar | Dihexose, Maltose, Lactose |
| H3-HNAc2-NANA | Sugar | Glucose |
| HNAC | Sugar | HNAC |
| 13S-HODE | Prostaglandine | 13S-Hydroxyoctadecadiensaure |
| 12S-HETE | Prostaglandine | 12S-Hydroxyeicosatetraensaure |
| 15S-HETE | Prostaglandine | 15S-Hydroxyeicosatetraensaure |
| TXB2 | Prostaglandine | Thromboxan B2 |
| DHA | Prostaglandine | Docosahexaenoic acid |
| PGE2 | Prostaglandine | Prostaglandin E2 |
| AA | Prostaglandine | Arachidonic Acid |
| KYNURENI | Biogenic Amine | Kynurenine |
| SPERMIDI | Biogenic Amine | Spermidine |
| PUTRESCI | Biogenic Amine | Putrescine |
| MET.SULF | Biogenic Amine | Met.Sulfoxide |
| SEROTONI | Biogenic Amine | Serotonine |
| ADMA | Biogenic Amine | Asymmetrical Dimethyl Arginine |
| total DMA | Biogenic Amine | total Dimethyl Arginine |
| SM (COOH) 18:0 | Sphingolipid | N-dicarboacyloysphingosylphosphocholine |
| SM (OH) C20:1 | Sphingolipid | N-hydroxyacyloysphingosylphosphocholine |
| SM (OH) C22:1 | Sphingolipid | N-hydroxyacyloysphingosylphosphocholine |
| SM (OH) C28:1 | Sphingolipid | N-hydroxyacyloysphingosylphosphocholine |
| SM (OH) C22:2 | Sphingolipid | N-hydroxyacyloysphingosylphosphocholine |
| SM (OH) C20:3 | Sphingolipid | N-hydroxyacyloysphingosyl -phosphocholine |
| SM (OH,COOH) C16:0 | Sphingolipid | N-hydroxydicarboacyloysphingosylphosphocholine |
| SM (OH,COOH) C20:3 | Sphingolipid | N-hydroxydicarboacyloysphingosylphosphocholine |
| SM (OH,COOH) C20:4 | Sphingolipid | N-hydroxydicarboacyloysphingosylphosphocholine |
| SM (COOH) 18:1 | Sphingolipid | N-dicarboacyloysphingosylphosphocholine |
| SM C14:0 | Sphingolipid | Sphingomyelin |
| SM C16:0 | Sphingolipid | Sphingomyelin |
| SM C18:0 (SM (OH,COOH) C14:2) | Sphingolipid | Sphingomyelin |
| SM C22:0 | Sphingolipid | Sphingomyelin |
| SM C24:0 | Sphingolipid | Sphingomyelin |
| SM C16:1 | Sphingolipid | Sphingomyelin |
| SM C18:1 | Sphingolipid | Sphingomyelin |
| SM C20:1 | Sphingolipid | Sphingomyelin |
| SM C22:1 | Sphingolipid | Sphingomyelin |
| SM C24:1 | Sphingolipid | Sphingomyelin |
| SM C20:2 | Sphingolipid | Sphingomyelin |
| SM C22:2 | Sphingolipid | Sphingomyelin |
| SM C24:2 | Sphingolipid | Sphingomyelin |
| SM (OH,COOH) C22:0 | Sphingolipid | N-hydroxydicarboacyloysphingosylphosphocholine |
| SM (OH,COOH) C24:0 | Sphingolipid | N-hydroxydicarboacyloysphingosylphosphocholine |
| SM (OH,COOH) C14:1 | Sphingolipid | N-hydroxydicarboacyloysphingosylphosphocholine |
| SM (OH,COOH) C16:1 | Sphingolipid | N-hydroxydicarboacyloysphingosylphosphocholine |
| SM (OH,COOH) C18:1 | Sphingolipid | N-hydroxydicarboacyloysphingosylphosphocholine |
| SM (OH,COOH) C24:1 | Sphingolipid | N-hydroxydicarboacyloysphingosylphosphocholine |
| SM (OH,COOH) C16:2 (SM | Sphingolipid | N-hydroxydicarboacyloysphingosyl- |
| C20:0) | | phosphocholine |
| SM (OH,COOH) C18:2 | Sphingolipid | N-hydroxydicarboacyloysphingosylphosphocholine |
| SM (OH,COOH) C20:2 | Sphingolipid | N-hydroxydicarboacyloysphingosylphosphocholine |
| SM C20:3 | Sphingolipid | Sphingomyelin |
| SM C24:3 | Sphingolipid | Sphingomyelin |
| SM C26:3 | Sphingolipid | Sphingomyelin |
| SM C28:3 | Sphingolipid | Sphingomyelin |
| SM C22:4 | Sphingolipid | Sphingomyelin |
| SM C24:4 | Sphingolipid | Sphingomyelin |
| SM C26:4 | Sphingolipid | Sphingomyelin |
| SM C28:4 | Sphingolipid | Sphingomyelin |
| SM (OH) C20:0 | Sphingolipid | N-hydroxyacyloysphingosylphosphocholine |
| SM (OH) C22:0 | Sphingolipid | N-hydroxyacyloysphingosylphosphocholine |
| SM (OH) C26:0 | Sphingolipid | N-hydroxyacyloysphingosyl- |
| | | phosphocholine |
| SM (OH) C28:0 | Sphingolipid | N-hydroxyacyloysphingosylphosphocholine |
| SM (COOH) C12:0 | Sphingolipid | N-dicarboacyloysphingosylphosphocholine |
| SM (COOH) C16:0 | Sphingolipid | N-dicarboacyloysphingosylphosphocholine |
| SM (COOH) C18:0 | Sphingolipid | N-dicarboacyloysphingosylphosphocholine |
| SM (COOH) C16:1 | Sphingolipid | N-dicarboacyloysphingosylphosphocholine |
| SM (COOH) C18:1 | Sphingolipid | N-dicarboacyloysphingosylphosphocholine |
| SM (COOH) C16:2 | Sphingolipid | N-dicarboacyloysphingosylphosphocholine |
| SM (COOH) C18:2 | Sphingolipid | N-dicarboacyloysphingosylphosphocholine |
| SM (COOH) C18:3 | Sphingolipid | N-dicarboacyloysphingosylphosphocholine |
| SM (OH) C24:2 | Sphingolipid | N-hydroxyacyloysphingosylphosphocholine |
| SM C22:3 | Sphingolipid | Sphingomyelin |
| SM (OH) C24:0 | Sphingolipid | N-hydroxyacyloysphingosylphosphocholine |
| GalCer sulf C24:2 | Sphingolipid | Galactosylceramide |
| SM C28:0 | Sphingolipid | Sphingomyelin |
| SM (OH,COOH) C20:1 | Sphingolipid | N-hydroxydicarboacyloysphingosylphosphocholine |
| SM (OH,COOH) C20:0 | Sphingolipid | N-hydroxydicarboacyloysphingosylphosphocholine |
| Cer (OH) C38:0 | Sphingolipid | Ceramide |
| Cer (OH) C16:1 | Sphingolipid | Ceramide |
| SM C20:4 | Sphingolipid | Sphingomyelin |
| SM C24:4 | Sphingolipid | Sphingomyelin |
| Cer 1 P C26:3 | Sphingolipid | Ceramide |
| Cer 1 P C26:4 | Sphingolipid | Ceramide |
| GalCer sulf (OH, COOH) | Sphingolipid | Galactosylceramide |
| C14:2 | | |
| Cer (COOH) C14:1 | Sphingolipid | Ceramide |
| GalCer sulf (OH, COOH) C14:3 | Sphingolipid | Galactosylceramide |
| GalCer sulf (OH) C14:3 | Sphingolipid | Galactosylceramide |
| SM C16:3 | Sphingolipid | Sphingomyelin |
| SM (OH) C14:1 | Sphingolipid | N-hydroxyacyloysphingosylphosphocholine |
| GalCer sulf C14:0 | Sphingolipid | Galactosylceramide |
| GalCer sulf C18:0 | Sphingolipid | Galactosylceramide |
| SM (OH,COOH) C12:0 | Sphingolipid | N-hydroxydicarboacyloysphingosylphosphocholine |
| SM (OH,COOH) C18:0 | Sphingolipid | N-hydroxydicarboacyloysphingosylphosphocholine |
| SM C12:0 | Sphingolipid | Sphingomyelin |
| SM (OH) C16:1 (SM (COOH) C14:1) | Sphingolipid | N-hydroxyacyloysphingosylphosphocholine |
| SM (OH,COOH) C6:0 | Sphingolipid | N-hydroxydicarboacyloysphingosylphosphocholine |
| SM (OH) C26:1 | Sphingolipid | N-hydroxyacyloysphingosylphosphocholine |
| SM (COOH) C14:0 | Sphingolipid | N-dicarboacyloysphingosylphosphocholine |
| SM (COOH) C14:1 | Sphingolipid | N-dicarboacyloysphingosylphosphocholine |
| SM (COOH) C14:2 | Sphingolipid | N-dicarboacyloysphingosylphosphocholine |
| Cer C20:3 (GlcCer C8:0) | Sphingolipid | Ceramide |
| Cer C24:3 | Sphingolipid | Ceramide |
| GlcCer C22:2 | Sphingolipid | Glucosylceramide |
| PI aa (COOH) C14:1 (PI aa | Glycerophospholipid | Phosphatidylinositol |
| (OH) C16:1, PI a C18:0, PI a | | |
| (OH, COOH) C14:2) | | |
| PIP aa C32:1 (PI aa C38:3) | Glycerophospholipid | Phosphatidylinositol phosphate |
| PC aa (COOH) C14:2 | Glycerophospholipid | Phoshatidylcholine |
| PC aa (COOH) C26:2 (PC a | Glycerophospholipid | Phoshatidylcholine |
| C30:1) | | |
| PC aa (COOH) C30:2 | Glycerophospholipid | Phoshatidylcholine |
| PC aa (COOH) C14:3 | Glycerophospholipid | Phoshatidylcholine |
| PC aa (COOH) C30:3 | Glycerophospholipid | Phoshatidylcholine |
| PC aa (COOH) C16:4 | Glycerophospholipid | Phoshatidylcholine |
| PC aa (COOH) C30:4 | Glycerophospholipid | Phoshatidylcholine |
| PC ae (OH) C20:0 (PC ae | Glycerophospholipid | Phoshatidylcholine |
| C22:6) | | |
| PC ae (OH) C28:1 | Glycerophospholipid | Phoshatidylcholine |
| PS aa (OH, COOH) C24:2 | Glycerophospholipid | Phosphatidylserine |
| PE e (COOH) C12:1 (PE ae | Glycerophospholipid | Phosphatidylethanolamine |
| C14:0, PE a C14:0, PE e (OH) | | |
| C14:1, PE e (COOH) C12:1) | | |
| PE e (COOH) C16:3 (PE e | Glycerophospholipid | Phosphatidylethanolamine |
| (COOH) C16:3, PE e (OH) | | |
| C18:3, PE ae (OH, COOH) | | |
| C14:4, PE a C18:2) | | |
| PC ae (OH, COOH) C30:3 | Glycerophospholipid | Phoshatidylcholine |
| PC ae (OH, COOH) C30:4 | Glycerophospholipid | Phoshatidylcholine |
| PC ae (COOH) C30:3 | Glycerophospholipid | Phoshatidylcholine |
| PC e C32:2 (PC aa C30:2, PC | Glycerophospholipid | Phoshatidylcholine |
| aa (OH, COOH) C26:4, PC ae | | |
| (OH) C30:3, PC ae (COOH) | | |
| C28:3) | | |
| PC aa C32:0 (PC ae C34:7, | Glycerophospholipid | Phoshatidylcholine |
| PC ae (COOH) C30:1, PC aa | | |
| (OH, COOH) C28:2) | | |
| PC aa C34:0 (PC ae C36:7, | Glycerophospholipid | Phoshatidylcholine |
| PC aa (OH, COOH) C30:2) | | |
| PC aa C36:0 (PC ae C38:7) | Glycerophospholipid | Phoshatidylcholine |
| PC aa C38:0 (PC ae C40:7) | Glycerophospholipid | Phoshatidylcholine |
| PC aa C20:1 (PC a (COOH) | Glycerophospholipid | Phoshatidylcholine |
| C18:2, PC e C22:1, PC ae | | |
| C22:8, PC aa (OH, COOH) | | |
| C16:3, PC ae (COOH) C18:2, | | |
| PC ae (OH) C20:2, PC e (OH, | | |
| COOH) C18:3) | | |
| PC aa C26:1 (PC e C28:1, PC | Glycerophospholipid | Phoshatidylcholine |
| ae C28:8, PC ae (OH) C26:2, | | |
| PC aa (OH, COOH) C22:3, PC | | |
| ae (COOH) C24:2) | | |
| PC aa C32:1 (PC ae (COOH) | Glycerophospholipid | Phoshatidylcholine |
| C30:2, PC aa (OH, COOH) | | |
| C28:3, PC ae C34:8) | | |
| PC aa C34:1 (PC ae C36:8) | Glycerophospholipid | Phoshatidylcholine |
| PC aa C36:1 (PC ae C38:8) | Glycerophospholipid | Phoshatidylcholine |
| PC aa C38:1 (PC ae C40:8) | Glycerophospholipid | Phoshatidylcholine |
| PE a (OH) C12:1 (PE e C14:0, | Glycerophospholipid | Phosphatidylethanolamine |
| PE aa C12:0) | | |
| PC e (COOH) C14:0 | Glycerophospholipid | Phoshatidylcholine |
| PC e (COOH) C12:1 | Glycerophospholipid | Phoshatidylcholine |
| PC e (COOH) C14:1 (PC ae | Glycerophospholipid | Phoshatidylcholine |
| C16:0) | | |
| PC e (COOH) C16:1 (PC ae | Glycerophospholipid | Phoshatidylcholine |
| (OH, COOH) C14:2, PC aa | | |
| (COOH) C14:1, PC ae C18:0) | | |
| PC aa C32:2 | Glycerophospholipid | Phoshatidylcholine |
| PC aa C34:2 | Glycerophospholipid | Phoshatidylcholine |
| PC aa C36:2 | Glycerophospholipid | Phoshatidylcholine |
| PC aa C38:2 | Glycerophospholipid | Phoshatidylcholine |
| PC aa C34:3 | Glycerophospholipid | Phoshatidylcholine |
| PC aa C36:3 | Glycerophospholipid | Phoshatidylcholine |
| PC aa C38:3 | Glycerophospholipid | Phoshatidylcholine |
| PC aa C34:4 | Glycerophospholipid | Phoshatidylcholine |
| PC aa C36:4 | Glycerophospholipid | Phoshatidylcholine |
| PC aa C38:4 | Glycerophospholipid | Phoshatidylcholine |
| PC aa C40:4 | Glycerophospholipid | Phoshatidylcholine |
| PC aa C36:5 | Glycerophospholipid | Phoshatidylcholine |
| PC aa C38:5 | Glycerophospholipid | Phoshatidylcholine |
| PC aa C40:5 | Glycerophospholipid | Phoshatidylcholine |
| PC aa C38:6 | Glycerophospholipid | Phoshatidylcholine |
| PC aa C40:6 | Glycerophospholipid | Phoshatidylcholine |
| PC aa (OH, COOH) C24:1 (PC | Glycerophospholipid | Phoshatidylcholine |
| ae (COOH) C26:0, PC ae (OH) | | |
| C28:0, PC ae C30:6) | | |
| PC aa (OH, COOH) C30:3 | Glycerophospholipid | Phoshatidylcholine |
| PC aa (OH, COOH) C28:4 | Glycerophospholipid | Phoshatidylcholine |
| PC aa (OH, COOH) C30:4 | Glycerophospholipid | Phoshatidylcholine |
| PC aa (OH) C14:0 (PC e (OH) | Glycerophospholipid | Phoshatidylcholine |
| C16:0) | | |
| PC ae C32:0 (PC ae (OH, | Glycerophospholipid | Phoshatidylcholine |
| COOH) C28:2, PC aa C32:7, | | |
| PC a C32:0, PC aa (OH) | | |
| C30:1, PC aa (COOH) C28:1) | | |
| PC ae C34:0 (PC aa C34:7, | Glycerophospholipid | Phoshatidylcholine |
| PC aa (COOH) C30:1, PC ae | | |
| (OH, COOH) C30:2) | | |
| PC ae C36:0 (PC aa C36:7) | Glycerophospholipid | Phoshatidylcholine |
| PC ae C38:0 (PC aa C38:7) | Glycerophospholipid | Phoshatidylcholine |
| PC ae C40:0 (PC aa C40:7) | Glycerophospholipid | Phoshatidylcholine |
| PC ae C32:1 (PC aa C32:8, | Glycerophospholipid | Phoshatidylcholine |
| PC a C32: 1, PC aa (OH) | | |
| C30:2, PC ae (OH, COOH) | | |
| C28:3, PC aa (COOH) C28:2) | | |
| PC ae C34:1 (PC aa C34:8) | Glycerophospholipid | Phoshatidylcholine |
| PC ae C36:1 (PC aa C36:8) | Glycerophospholipid | Phoshatidylcholine |
| PC ae C38:1 (PC aa C38:8) | Glycerophospholipid | Phoshatidylcholine |
| PC ae C40:1 (PC aa C40:8) | Glycerophospholipid | Phoshatidylcholine |
| PC ae C34:2 | Glycerophospholipid | Phoshatidylcholine |
| PC ae C36:2 | Glycerophospholipid | Phoshatidylcholine |
| PC ae C38:2 | Glycerophospholipid | Phoshatidylcholine |
| PC ae C40:2 | Glycerophospholipid | Phoshatidylcholine |
| PC ae C20:3 (PC a C20:3, PC | Glycerophospholipid | Phoshatidylcholine |
| aa (OH) C18:4) | | |
| PC ae C34:3 | Glycerophospholipid | Phoshatidylcholine |
| PC ae C36:3 | Glycerophospholipid | Phoshatidylcholine |
| PC ae C38:3 | Glycerophospholipid | Phoshatidylcholine |
| PC ae C40:3 | Glycerophospholipid | Phoshatidylcholine |
| PC ae C36:4 | Glycerophospholipid | Phoshatidylcholine |
| PC ae C38:4 | Glycerophospholipid | Phoshatidylcholine |
| PC ae C40:4 | Glycerophospholipid | Phoshatidylcholine |
| PC ae C36:5 (PC aa (OH, | Glycerophospholipid | Phoshatidylcholine |
| COOH) C30:0) | | |
| PC ae C38:5 | Glycerophospholipid | Phoshatidylcholine |
| PC ae C40:5 | Glycerophospholipid | Phoshatidylcholine |
| PC ae C42:5 | Glycerophospholipid | Phoshatidylcholine |
| PC ae C44:5 | Glycerophospholipid | Phoshatidylcholine |
| PC ae C34:6 (PC ae (COOH) | Glycerophospholipid | Phoshatidylcholine |
| C30:0, PC aa (OH, COOH) | | |
| C28: 1) | | |
| PC ae C36:6 (PC aa (OH, | Glycerophospholipid | Phoshatidylcholine |
| COOH) C30:1) | | |
| PC ae C38:6 | Glycerophospholipid | Phoshatidylcholine |
| PC ae C40:6 | Glycerophospholipid | Phoshatidylcholine |
| PC ae C42:6 | Glycerophospholipid | Phoshatidylcholine |
| PC ae C32:7 (PC ae (COOH) | Glycerophospholipid | Phoshatidylcholine |
| C28:1, PC ae (OH) C30:1, PC | | |
| aa C30:0, PC aa (OH, COOH) | | |
| C26:2, PC e C32:0, PC ae | | |
| C32:7) | | |
| PC ae C32:8 (PC ae (OH) | Glycerophospholipid | Phoshatidylcholine |
| C30:2, PC e C32:1, PC ae | | |
| C32:8, PC ae (COOH) C28:2, | | |
| PC aa C30:1, PC aa (OH, | | |
| COOH) C26:3) | | |
| PC a C16:1 (PC e (OH) C16:2) | Glycerophospholipid | Phoshatidylcholine |
| PC a C18:1 (PC e (COOH) | Glycerophospholipid | Phoshatidylcholine |
| C16:2, PC ae (OH, COOH) | | |
| C14:3, PC ae C18:1, PC aa | | |
| (OH) C16:2, PC e (OH) C18:2, | | |
| PC a C18:1) | | |
| PE a (OH, COOH) C12:2 | Glycerophospholipid | Phosphatidylethanolamine |
| PE a (OH, COOH) C14:3 (PE e | Glycerophospholipid | Phosphatidylethanolamine |
| (COOH) C16:2) | | |
| PC a C18:2 (PC e (OH) C18:3, | Glycerophospholipid | Phoshatidylcholine |
| PC e (COOH) C16:3, PC ae | | |
| (OH, COOH) C14:4, PC ae | | |
| C18:2, PC aa (OH) C16:3) | | |
| PC a C32:2 | Glycerophospholipid | Phoshatidylcholine |
| PC a C20:4 (PC ae C20:4) | Glycerophospholipid | Phoshatidylcholine |
| PC e C18:0 (PC a (COOH) | Glycerophospholipid | Phoshatidylcholine |
| C14:1, PC a C17:0, PC a (OH) | | |
| C16:1) | | |
| PC e C30:0 (PC aa (OH, | Glycerophospholipid | Phoshatidylcholine |
| COOH) C24:2, PC ae C30:7) | | |
| PC e (OH) C14:1 (PC a C14:0) | Glycerophospholipid | Phoshatidylcholine |
| PE aa C34:2 | Glycerophospholipid | Phosphatidylethanolamine |
| PE aa C36:2 | Glycerophospholipid | Phosphatidylethanolamine |
| PE aa C36:4 | Glycerophospholipid | Phosphatidylethanolamine |
| PE aa C38:4 | Glycerophospholipid | Phosphatidylethanolamine |
| PE aa C38:5 | Glycerophospholipid | Phosphatidylethanolamine |
| PE aa C38:6 | Glycerophospholipid | Phosphatidylethanolamine |
| PE aa C40:6 | Glycerophospholipid | Phosphatidylethanolamine |
| PA aa C22:2 | Glycerophospholipid | Phosphatidic Acid |
| PG a C26:6 | Glycerophospholipid | Phosphatidylglycerol |
| PE ae C42:7 | Glycerophospholipid | Phosphatidylethanolamine |
| PE a C10:0 | Glycerophospholipid | Phosphatidylethanolamine |
| PS ae C42:6 | Glycerophospholipid | Phosphatidylserine |
| PA aa C42:5 | Glycerophospholipid | Phosphatidic Acid |
| PA aa C20:7 | Glycerophospholipid | Phosphatidic Acid |
| PA a C20:1 | Glycerophospholipid | Phosphatidic Acid |
| PI aa (OH, COOH) C28:1 | Glycerophospholipid | Phosphatidylinositol |
| PI a (OH, COOH) C12:2 (PI aa | Glycerophospholipid | Phosphatidylinositol |
| (OH) C14:1, PI a C16:0) | | |
| PI aa C34:1 (PI aa (OH, | Glycerophospholipid | Phosphatidylinositol |
| COOH) C30:3) | | |
| PI aa C36:1 | Glycerophospholipid | Phosphatidylinositol |
| PI aa C36:2 (PIP aa C30:0) | Glycerophospholipid | Phosphatidylinositol |
| PI aa C16:3 | Glycerophospholipid | Phosphatidylinositol |
| PI aa C36:4 (PIP aa C30:2, | Glycerophospholipid | Phosphatidylinositol |
| PIP2 aa C24:0) | | |
| PI aa C38:4 (PIP aa C32:2, | Glycerophospholipid | Phosphatidylinositol |
| PIP2 aa C26:0) | | |
| PC ae C22:8 | Glycerophospholipid | Phoshatidylcholine |
| PC ae C30:1 (PC aa (OH) | Glycerophospholipid | Phoshatidylcholine |
| C28:2, PC a C30:1, PC aa | | |
| C30:8, PC aa (COOH) C26:2) | | |
| PC ae (OH, COOH) C28:2 | Glycerophospholipid | Phoshatidylcholine |
| PC ae (OH, COOH) C30:2 | Glycerophospholipid | Phoshatidylcholine |
| PC ae (OH, COOH) C28:3 | Glycerophospholipid | Phoshatidylcholine |
| PIP2 aa C24:0 | Glycerophospholipid | Phosphatidylinositol bisphosphate |
| PIP2 aa C26:0 | Glycerophospholipid | Phosphatidylinositol bisphosphate |
| PI aa C20:7 (PI aa (COOH) | Glycerophospholipid | Phosphatidylinositol |
| C16:1, PI aa (OH) C18:1, PI a | | |
| C20:0, PI a (OH, COOH) | | |
| C16:2) | | |
| PI aa C38:3 | Glycerophospholipid | Phosphatidylinositol |
| PI e C14:2 (PIP aa C6:0) | Glycerophospholipid | Phosphatidylinositol |
| PIP aa C28:0 (PI aa C34:2) | Glycerophospholipid | Phosphatidylinositol phosphate |
| PI a C18:0 | Glycerophospholipid | Phosphatidylinositol |
| PIP aa C30:2 | Glycerophospholipid | Phosphatidylinositol phosphate |
| PIP aa C32:2 | Glycerophospholipid | Phosphatidylinositol phosphate |
| PI aa (OH, COOH) C28:2 | Glycerophospholipid | Phosphatidylinositol |
| PI a (OH) C12:3 | Glycerophospholipid | Phosphatidylinositol |
| PI a (OH, COOH) C14:2 | Glycerophospholipid | Phosphatidylinositol |
| PIP aa C14:3 | Glycerophospholipid | Phosphatidylinositol phosphate |
| PI aa (OH) C16:1 | Glycerophospholipid | Phosphatidylinositol |
| PA a (OH) C28:1 | Glycerophospholipid | Phosphatidic Acid |
| PC e C28:1 (PC ae C28:8, PC | Glycerophospholipid | Phoshatidylcholine |
| e C28:1, PC ae (OH) C26:2, | | |
| PC aa C26:1, PC ae (COOH) | | |
| C24:2, PC aa (OH, COOH) | | |
| C22:3) | | |
| PC aa (OH, COOH) C24:2 | Glycerophospholipid | Phoshatidylcholine |
| PE a (COOH) C12:4 (PE e | Glycerophospholipid | Phosphatidylethanolamine |
| C16:3) | | |
| PC a C20:3 (PC ae C20:3, PC | Glycerophospholipid | Phoshatidylcholine |
| aa (OH) C18:4, PC aa (COOH) | | |
| C16:4) | | |
| PS aa C28:3 | Glycerophospholipid | Phosphatidylserine |
| PE a (OH) C10:0 (PE a | Glycerophospholipid | Phosphatidylethanolamine |
| (COOH) C8:0) | | |
| PG a (OH, COOH) C14:3 | Glycerophospholipid | Phosphatidylglycerol |
| PC a C22:6 (PC a (COOH) | Glycerophospholipid | Phoshatidylcholine |
| C18:0) | | |
| PC e C10:0 (PC a C9:0, PC aa | Glycerophospholipid | Phoshatidylcholine |
| C8:0) | | |
| PC a (OH) C16:1 (PC a C17:0, | Glycerophospholipid | Phoshatidylcholine |
| PC e C18:0) | | |
| PE e C16:2 (PE a (OH) C14:3, | Glycerophospholipid | Phosphatidylethanolamine |
| PE a (COOH) C12:3, PE ae | | |
| (OH) C14:3, PE e (OH, COOH) | | |
| C12:4) | | |
| PC a (COOH) C18:0 (PC a | Glycerophospholipid | Phoshatidylcholine |
| C22:6, PC ae (COOH) C18:0, | | |
| PC ae C22:6, PC ae (OH) | | |
| C20:0) | | |
| PC ae C42:3 | Glycerophospholipid | Phoshatidylcholine |
| PE e (OH, COOH) C12:4 (PE e | Glycerophospholipid | Phosphatidylethanolamine |
| C16:2) | | |
| PC aa C40:3 | Glycerophospholipid | Phoshatidylcholine |
| PC ae C44:6 | Glycerophospholipid | Phoshatidylcholine |
| PE e C14:0 | Glycerophospholipid | Phosphatidylethanolamine |
| PE aa C38:3 | Glycerophospholipid | Phosphatidylethanolamine |
| PE aa C28:0 | Glycerophospholipid | Phosphatidylethanolamine |
| PE e C30:0 (PE ae C30:7, PE | Glycerophospholipid | Phosphatidylethanolamine |
| ae (OH) C28:1, PE aa (OH, | | |
| COOH) C24:2, PE ae (COOH) | | |
| C26:1) | | |
| PI aa C34:2 (PIP aa C28:0, PI | Glycerophospholipid | Phosphatidylinositol |
| aa (OH, COOH) C30:4) | | |
| PC ae C30:8 (PC aa C28:1) | Glycerophospholipid | Phoshatidylcholine |
| PC e (OH) C16:0 | Glycerophospholipid | Phoshatidylcholine |
| PC e C22:1 (PC a (COOH) | Glycerophospholipid | Phoshatidylcholine |
| C18:2, PC e C22:1, PC ae | | |
| C22:8, PC ae (COOH) C18:2, | | |
| PC aa (OH, COOH) C16:3, PC | | |
| aa C20:1, PC ae (OH) C20:2, | | |
| PC e (OH, COOH) C18:3) | | |
| PE aa C40:5 | Glycerophospholipid | Phosphatidylethanolamine |
| PC aa C36:6 | Glycerophospholipid | Phoshatidylcholine |
| PE a (COOH) C10:0 | Glycerophospholipid | Phosphatidylethanolamine |
| PE e (COOH) C14:1 | Glycerophospholipid | Phosphatidylethanolamine |
| PE e (COOH) C16:1 | Glycerophospholipid | Phosphatidylethanolamine |
| PE e (COOH) C16:2 | Glycerophospholipid | Phosphatidylethanolamine |
| PE a (OH) C12:0 | Glycerophospholipid | Phosphatidylethanolamine |
| PE ae (OH) C28:1 | Glycerophospholipid | Phosphatidylethanolamine |
| PE ae (OH) C14:4 | Glycerophospholipid | Phosphatidylethanolamine |
| PC a C26:1 | Glycerophospholipid | Phoshatidylcholine |
| PE aa (COOH) C14:1 | Glycerophospholipid | Phosphatidylethanolamine |
| PE ae (OH, COOH) C14:2 | Glycerophospholipid | Phosphatidylethanolamine |
| PE ae C30:7 | Glycerophospholipid | Phosphatidylethanolamine |
| PE e (OH) C14:1 | Glycerophospholipid | Phosphatidylethanolamine |
| PE e (OH) C18:1 | Glycerophospholipid | Phosphatidylethanolamine |
| PE ae (COOH) C26:1 | Glycerophospholipid | Phosphatidylethanolamine |
| PC ae C42:2 | Glycerophospholipid | Phoshatidylcholine |
| PC ae C36:8 | Glycerophospholipid | Phoshatidylcholine |
| PC ae C38:8 | Glycerophospholipid | Phoshatidylcholine |
| PC a C30:0 | Glycerophospholipid | Phoshatidylcholine |
| PE a C22:6 | Glycerophospholipid | Phosphatidylethanolamine |
| PI a (OH, COOH) C18:2 (PI aa | Glycerophospholipid | Phosphatidylinositol |
| (COOH) C18:1, PI aa (OH) | | |
| C20:1, PI a C22:0, PI aa | | |
| C22:7, PI a (OH, COOH) | | |
| C18:2) | | |
| PG aa (OH, COOH) C20:2 | Glycerophospholipid | Phosphatidylglycerol |
| PG e (OH) C12:2 | Glycerophospholipid | Phosphatidylglycerol |
| PE e C16:3 (PE aa C14:3) | Glycerophospholipid | Phosphatidylethanolamine |
| PE a (OH, COOH) C14:2 (PE a | Glycerophospholipid | Phosphatidylethanolamine |
| C18:0) | | |
| PI aa (OH) C14:1 | Glycerophospholipid | Phosphatidylinositol |
| PIP aa C6:0 (PI e C14:2) | Glycerophospholipid | Phosphatidylinositol phosphate |
| PC ae C32:6 (PC aa (OH, | Glycerophospholipid | Phoshatidylcholine |
| COOH) C26:1, PC a C32:6, PC | | |
| ae (OH) C30:0) | | |
| PI a C14:2 | Glycerophospholipid | Phosphatidylinositol |
| PC aa (OH, COOH) C22:2 (PC | Glycerophospholipid | Phoshatidylcholine |
| aa C26:0) | | |
| PE aa (OH, COOH) C24:2 | Glycerophospholipid | Phosphatidylethanolamine |
| PI a C16:0 (PI a (OH, COOH) | Glycerophospholipid | Phosphatidylinositol |
| C12:2) | | |
| PC ae C32:2 (PC ae C32:2, | Glycerophospholipid | Phoshatidylcholine |
| PC aa (OH) C30:3, PC a | | |
| C32:2) | | |
| PC ae C44:4 | Glycerophospholipid | Phoshatidylcholine |
| PC a (OH) C14:1 (PC e C16:0, | Glycerophospholipid | Phoshatidylcholine |
| PC a (COOH) C12:1) | | |
| PC aa C42:6 | Glycerophospholipid | Phoshatidylcholine |
| PC a C30:1 (PC aa (OH) | Glycerophospholipid | Phoshatidylcholine |
| C28:2, PC ae (OH, COOH) | | |
| C26:3, PC ae C30:1, PC aa | | |
| (COOH) C26:2, PC aa C30:8) | | |
| PC a C20:5 | Glycerophospholipid | Phoshatidylcholine |
| PC a C22:5 | Glycerophospholipid | Phoshatidylcholine |
| PC ae C42:4 | Glycerophospholipid | Phoshatidylcholine |
| PE aa C34:1 | Glycerophospholipid | Phosphatidylethanolamine |
| PE aa C36:3 | Glycerophospholipid | Phosphatidylethanolamine |

**Table 4: Genetically determined metabotypes with the strongest signal of association. Reported are the SNP identifier (rs number), chromosome, chromosomal position, the minor allele frequency (MAF), the metabolic trait with the lowest p-value of association (test against the null-hypothesis of no association), and percentage of the variance explained by the additive genetic model. Association results for metabolic traits with p<0.05 are provided in Tables 2 and S2 to S5. Data for all 363 metabolic traits are available as supporting online data. P-values of association from previous GWA studies for the same SNP (neighboring SNP rs1200826 for ANKRD30A) are reported for the following traits: (a) HDL cholesterol, LDL cholesterol, triglycerides are from the publication of Willer et al. (2008); (b) 2h glucose, 2h insulin, apolipoproteins A-I, A-II, B, total cholesterol, fasting glucose, fasting insulin, HOMA insulin resistance, insulinogenic index are from the Diabetes Genetics Initiative (DGI) study (Kathiresan et al. 2008); (c) bipolar disorder, coronary artery disease, Crohn's disease, hypertension, rheumatoid arthritis, type 1 and type 2 diabetes mellitus are from the WTCCC study (2007). Associations with p-values larger than 0.1 are indicated by a '-'.**

| Gene | *PLEK* | *PARK2* | *ANKRD30A* | *FADS1* | *LIPC* |
|---|---|---|---|---|---|
| Position relative to gene | 21 kb upstream | intron | 3'UTR | intron | 49kb upstream |
| rs number | rs9309413 | rs992037 | rs1148259 (rs1200826) | rs174548 | rs4775041 |
| Chromosome | 2 | 6 | 10 | 11 | 15 |
| Chromosomal | 68,482,423 | 161,971,847 | 37,548,456 | 61,327,924 | 56,461,987 |
| position | | | | | |
| Minor allele | 45.2% | 34.7% | 42.2% | 27.5% | 28.0% |
| frequency | | | | | |
| Best metabolic trait | Sphingomyelin SM C14:0 | Lysine | Sphingomyelin SM(OH,COOH) C18:2 | Phosphatidylcholine PC aa C36:4 | Phosphatidylethanolamine PE aa C38:6 |
| P-value of best metabolic trait | 1.95x10⁻⁹ | 1.20x10⁻⁷ | 3.04x10⁻⁹ | 4.52x10⁻⁸ | 9.66x10⁻⁸ |
| Explained variance | 12.0% | 9.5% | 11.7% | 10.1% | 9.7% |

| **Traits in GWAS** | | | | | |
|---|---|---|---|---|---|
| HDL cholesterol^{a} | 0.035 | - | - | 1.89x10⁻⁴ | 2.80x10⁻⁹ |
| LDL cholesterol^{a} | - | - | - | - | - |
| Triglycerides^{a} | - | - | - | 0.0014 | 7.30x10⁻⁵ |
| 2h glucose^{b} | - | - | - | - | - |
| 2h insulin^{b} | - | - | - | - | - |
| Apolipoprotein-I, APOA-1^{b} | - | - | 2.44x10⁻⁴ | 0.032 | 2.75x10⁻⁴ |
| Apolipoprotein-II, APOA-2^{b} | - | - | 0.033 | 0.0055 | 0.0032 |
| Apolipoprotein B, APOB^{b} | - | - | - | - | - |
| Total cholesterol^{b} | - | - | 0.043 | 1.48x10⁻⁴ | 0.055 |
| Fasting glucose^{b} | - | - | - | - | - |
| Fasting insulin^{b} | - | - | - | - | - |
| HDL cholesterol^{b} | - | - | - | 0.037 | 0.0049 |
| fasting insulin, HOMA^{b} | - | - | - | - | - |
| Insulinogenic index^{b} | - | - | - | - | 0.016 |
| LDL cholesterol^{b} | - | - | 0.058 | 6.07x10⁻⁵ | - |
| Triglycerides/HDL^{b} | 0.010 | - | - | 0.051 | 0.025 |
| Triglycerides^{b} | - | - | - | 0.028 | 0.0071 |
| Bipolar disorder^{c} | - | - | - | 0.048 | 0.046 |
| Coronary artery disease ^{c} | - | - | - | 0.021 | - |
| Crohn's disease^{c} | - | - | - | 0.027 | - |
| Hypertension^{c} | - | - | - | - | - |
| Rheumatoid arthritis^{c} | 0.031 | - | - | - | 0.059 |
| Type 1 diabetes mellitus^{c} | - | - | - | - | - |
| Type 2 diabetes mellitus^{c} | - | - | - | - | 0.061 |

**Table 5: List of all associations with a p-value of association smaller than 10⁻⁶ for at least one of the tested metabolic traits. Reported are the SNP identifier (rs number), its chromosome (Chr.) and its chromosomal position (Position), the minor allele frequency (MAF), and the metabolic trait with the lowest p-value of association (test against the null-hypothesis of no association); where an association (p<0.1) of the same SNP has been reported in one of the recent GWA studies (WTCCC 2007; Kathiresan et al. 2008; Willer et al. 2008), the p-value of the strongest association is reported in the comment column**

| **rs number** | **Chr.** | **Position** | **MAF** | **best metabolic trait** | **p-value** | **Comment** |
|---|---|---|---|---|---|---|
| rs9309413 | 2 | 68,482,423 | 45.2% | Sphingomyelin SM C14:0 | 1.95E-9 | 21 kb upstream PLEK (pleckstrin); triglyceride/HDL (p=0.010) |
| rs6807064 | 3 | 10,510,771 | 29.3% | Phenylalanine | 1.31E-07 | 44kb upstream ATP2B2 (ATPase, plasma membrane calcium pump); TG (p=0.023) |
| rs1382269 | 3 | 138,591,180 | 47.0% | Sphingomyelin SM (OH,COOH) C20:3 | 4.52E-07 | intergenic; LDL (p=0.078) |
| rs4453795 | 3 | 193,576,677 | 40.9% | Phosphatidylcholine PC aa C34:4 | 3.86E-07 | intron FGF12 (fibroblast growth factor 12); BD (p=0.059) |
| rs10517480 | 4 | 60,577,595 | 30.6% | Phosphatidylcholine PC ae C38:3 | 1.74E-07 | intergenic |
| rs9354308 | 6 | 66,622,074 | 36.7% | Serotonin | 3.12E-07 | intergenic; RA (p=0.044) |
| rs9342503 | 6 | 66,622,157 | 36.7% | Serotonin | 3.12E-07 | intergenic; RA (p=0.049) |
| rs9360161 | 6 | 66,622,178 | 36.7% | Serotonin | 3.12E-07 | intergenic; RA (p=0.040) |
| rs1591830 | 6 | 150,744,014 | 32.3% | Sugar H3-HNAc2-NANA | 1.28E-07 | 39kb upstream C6orf71 (iodotyrosine deiodinase); TG (p=0.0192) |
| rs992037 | 6 | 161,971,847 | 34.7% | Lysine | 1.20E-07 | intron PARK2 (parkin) |
| rs10953730 | 7 | 112,505,361 | 34.8% | Acylcarnitine C12 C4:1-DC C10 | 1.93E-07 2.54E-07 2.58E-07 | intergenic; T2D (p=0.0072) |
| rs1148259 | 10 | 37,548,456 | 42.2% | Sphingomyelin SM(OH,COOH) C18:2 | 3.04E-09 | 3'UTR ANKRD30A (ankyrin repeat domain 30A) |
| rs1200826 | 10 | 37,604,993 | 48.2% | Sphingomyelin SM(OH,COOH) C18:2 | 5.14E-08 | 43 kb downstream ANKRD30A (ankyrin repeat domain 30A); APOA1 (p=2.44x10⁻⁴) |
| rs7081443 | 10 | 94,997,483 | 47.2% | Phosphatidylethanoa mine PE a C10:0 | 4.27E-07 | 59kb downstream FER1 L3 (myoferlin); |
| rs12765326 | 10 | 95,004,928 | 47.2% | Phosphatidylethanoa mine PE a C10:0 | 2.34E-07 | TG (p=0.025) intergenic; TG (p=0.028) |
| rs174548 | 11 | 61,327,924 | 27.5% | Phosphatidylcholine PC aa C36:4 | 4.52E-08 | intron FADS1 (fatty acid desaturase 1); LDL (p=6.07x10⁻⁵) |
| rs174549 | 11 | 61,327,958 | 27.0% | Phosphatidylcholine PC aa C36:4 | 2.20E-07 | intron FADS1 (fatty acid desaturase 1); LDL (p=9.22x10⁻⁵) |
| rs174455 | 11 | 61,412,693 | 35.9% | Phosphatidylethanoa mine PE a C18:2 | 4.23E-07 | intron FADS3 (fatty acid desaturase 3); HDL (p=8.13x10⁻⁵) |
| rs2194980 | 12 | 113,965,438 | 33.2% | Tyrosine | 3.14E-07 | intergenic; 2h glucose (p=0.051) |
| rs17267292 | 13 | 92,121,147 | 27.7% | Docosahexaonic acid | 1.28E-07 | intron GPC5 (glypican 5); residual cholesterol (p=0.017) |
| rs4775041 | 15 | 56,461,987 | 28.0% | Phosphatidylethanoa mine PE aa C38:6 | 9.66E-08 | 49kb upstream LIPC (hepatic lipase); HDL (p=2.80x10⁻⁹) |
| rs756873 | 15 | 89,518,332 | 37.6% | Sphingomyelin SM (COOH) C18:3 | 1.42E-07 | 52kb upstream SV2B (synaptic vesicle glycoprotein 2B); APOB (p=0.052) |
| rs886144 | 15 | 89,518,356 | 37.6% | Sphingomyelin SM (COOH) C18:3 | 2.17E-07 | 52kb upstream SV2B (synaptic vesicle glycoprotein 2B); T1D (p=0.065) |
| rs9935875 | 16 | 7,444,597 | 41.2% | Phosphatidylcholine PC ae C34:2 | 4.57E-07 | intron A2BP1 (ataxin 2-binding protein 1) |
| rs9935962 | 16 | 7,444,671 | 41.3% | Phosphatidylcholine PC ae C34:2 | 3.93E-07 | intron A2BP1 (ataxin 2-binding protein 1) |
| rs9924951 | 16 | 7,444,855 | 40.6% | Phosphatidylcholine PC ae C34:2 | 4.97E-07 | intron A2BP1 (ataxin 2-binding protein 1) |
| rs9936248 | 16 | 7,445,030 | 41.5% | Phosphatidylcholine PC ae C34:2 | 4.70E-07 | intron A2BP1 (ataxin 2-binding protein 1); HDL (p=0.074) |

**Table 6: Associations of rs174548 (FADS1) with metabolic traits. Metabolites associated (p<0.05) with genotype rs174548 (FADS1) in the additive genetic model; in cases where alternative assignments of the metabolites are possible, these are indicated by a '*'. Full annotations can be found in the supplementary online data files. Reported are the mean concentrations (µM), standard deviation, the number of cases for which metabolite concentrations were obtained (ncases), the p-value of the association, the regression coefficient using an additive genetic model (estimate), and the measure of the observed variance that can be explained by the additive genetic model.**

| | | | | | |
|---|---|---|---|---|---|
| | | | | | |

| **metabolite** | **mean** | **ncases** | **p-value** | **estimate** | **explained variance** |
|---|---|---|---|---|---|
| PC aa C36:4 | 399.41 | 284 | 4.52E-08 | -0.318 | 10.11% |
| PC a C20:4* | 5.09 | 284 | 5.30E-07 | -0.293 | 8.58% |
| PC aa C38:4 | 209.05 | 284 | 4.91 E-06 | -0.268 | 7.17% |
| PC ae C36:5* | 19.14 | 284 | 1.46E-05 | -0.255 | 6.48% |
| SM C22:2 | 4.94 | 284 | 5.93E-05 | -0.236 | 5.59% |
| PC ae C38:4 | 30.12 | 284 | 1.42E-04 | -0.224 | 5.03% |
| PE aa C34:2 | 2.22 | 284 | 1.54E-04 | 0.223 | 4.98% |
| PC ae C38:5 | 32.72 | 284 | 1.80E-04 | -0.221 | 4.88% |
| PC aa C38:5 | 128.89 | 284 | 2.01 E-04 | -0.219 | 4.81% |
| PE e (COOH) C16:3* | 5.05 | 284 | 1.49E-03 | 0.188 | 3.53% |
| PC ae C36:4 | 35.16 | 284 | 1.68E-03 | -0.186 | 3.46% |
| PE a C10:0 | 4.16 | 284 | 2.34E-03 | -0.180 | 3.25% |
| PC aa C34:2 | 810.00 | 284 | 2.68E-03 | 0.178 | 3.16% |
| SM (COOH) C18:3 | 7.30 | 284 | 3.08E-03 | -0.175 | 3.07% |
| PC aa C34:4 | 3.25 | 284 | 3.25E-03 | -0.174 | 3.04% |
| PC aa C36:5 | 47.53 | 284 | 4.65E-03 | -0.168 | 2.82% |
| PC ae C36:2 | 25.33 | 284 | 5.87E-03 | 0.163 | 2.67% |
| PC aa C40:5 | 27.52 | 284 | 6.21 E-03 | -0.162 | 2.63% |
| Arachidonic acid | 4.33 | 283 | 9.04E-03 | -0.155 | 2.41% |
| PC ae C40:5 | 6.79 | 284 | 1.05E-02 | -0.152 | 2.31% |
| PC aa C40:4 | 9.53 | 284 | 1.07E-02 | -0.151 | 2.29% |
| SM (OH) C26:1 | 12.75 | 63 | 1.15E-02 | -0.317 | 10.03% |
| PI aa C36:2* | 7.37 | 284 | 1.15E-02 | 0.150 | 2.25% |
| SM C24:2 | 16.82 | 221 | 1.20E-02 | -0.169 | 2.86% |
| PI aa C38:4* | 27.03 | 284 | 1.21 E-02 | -0.149 | 2.22% |
| PC aa (OH, COOH) C30:4 | 342.95 | 284 | 1.29E-02 | 0.148 | 2.18% |
| PC ae C34:2 | 23.44 | 284 | 2.28E-02 | 0.135 | 1.83% |
| SM (OH) C24:0 | 11.80 | 208 | 2.94E-02 | -0.151 | 2.29% |
| LYS | 215.17 | 284 | 3.05E-02 | 0.129 | 1.65% |
| PA aa C20:7 | 197.36 | 284 | 3.20E-02 | -0.128 | 1.63% |
| PE aa C36:2 | 4.42 | 284 | 3.52E-02 | 0.125 | 1.57% |
| PC aa (COOH) C30:3* | 10.38 | 215 | 4.00E-02 | 0.141 | 1.98% |
| PC ae C38:6 | 11.67 | 284 | 4.40E-02 | -0.120 | 1.44% |
| PC aa C38:6 | 146.59 | 284 | 4.42E-02 | -0.120 | 1.43% |
| C5-DC | 0.11 | 284 | 4.43E-02 | -0.120 | 1.43% |
| SM (OH,COOH) C6:0 | 4.79 | 63 | 4.64E-02 | 0.252 | 6.35% |
| SM C28:4 | 5.51 | 284 | 4.73E-02 | -0.118 | 1.39% |
| PI a (OH, COOH) C18:2* | 3.74 | 63 | 4.84E-02 | 0.250 | 6.24% |
| PC aa C36:2 | 412.59 | 284 | 4.92E-02 | 0.117 | 1.37% |

**Table 7: Associations of rs174548 (FADS1) with concentrations and ratios between the concentrations of matching pairs of glycerophospholipid species. Association of SNP rs174548 (FADS1) with concentrations and ratios between the concentrations of matching pairs of glycerophospholipid species with three- (denominator) and four-fold (enumerator) unsaturated carbon bonds in their fatty acid side chains; in cases where alternative assignments of the metabolites are possible, these are indicated by a '*'; reported are the mean (µM), the number of cases for which metabolite concentrations were obtained (ncases), the p-value of the association, the regression coefficient using an additive genetic model (estimate), and the proportion of the observed variance that can be explained by including the genetic polymorphism in the additive genetic model.**

| **enumerator** | **denominator** | **mean** | **ncases** | **p-value** | **estimate** | **explained variance** |
|---|---|---|---|---|---|---|
| **Single metabolites (four double bonds)** | | | | | | |
| PC a C20:4* | 1 | 5.094 | 284 | 5.3x10⁻⁷ | -0.293 | 8.58% |
| PC aa C34:4 | 1 | 3.249 | 284 | 3.3x10⁻³ | -0.174 | 3.04% |
| PC aa C36:4 | 1 | 399.407 | 284 | 4.5x10⁻⁸ | -0.318 | 10.11% |
| PC aa C38:4 | 1 | 209.050 | 284 | 4.9x10⁻⁶ | -0.268 | 7.17% |
| PC ae C36:4 | 1 | 35.160 | 284 | 1.7x10⁻³ | -0.186 | 3.46% |
| PC ae C38:4 | 1 | 30.117 | 284 | 1.4x10⁻⁴ | -0.224 | 5.03% |
| PE aa C38:4 | 1 | 5.357 | 284 | 0.13 | -0.090 | 0.81% |
| PI aa C38:4* | 1 | 27.025 | 284 | 0.012 | -0.149 | 2.22% |
| **Single metabolites (three double bonds)** | | | | | | |
| PC a C20:3* | 1 | 2.461 | 208 | 0.86 | -0.013 | 0.02% |
| PC aa C34:3 | 1 | 30.751 | 284 | 0.21 | 0.075 | 0.56% |
| PC aa C36:3 | 1 | 250.496 | 284 | 0.56 | 0.035 | 0.12% |
| PC aa C38:3 | 1 | 123.002 | 284 | 0.66 | -0.027 | 0.07% |
| PC ae C36:3 | 1 | 19.697 | 284 | 0.17 | 0.081 | 0.66% |
| PC ae C38:3 | 1 | 10.641 | 284 | 0.74 | 0.020 | 0.04% |
| PE aa C38:3 | 1 | 1.623 | 132 | 0.92 | -0.009 | 0.01% |
| PI aa C38:3* | 1 | 7.791 | 221 | 0.077 | 0.120 | 1.43% |
| **Ratios between metabolite concentrations** | | | | | | |
| PC a C20:4* | PC a C20:3* | 2.224 | 208 | 2.9x10⁻⁸ | -0.374 | 13.98% |
| PC aa C34:4 | PC aa C34:3 | 0.107 | 284 | 4.2x10⁻⁷ | -0.295 | 8.72% |
| PC aa C36:4 | PC aa C36:3 | 1.613 | 284 | 2.4x10⁻²² | -0.535 | 28.62% |
| PC aa C38:4 | PC aa C38:3 | 1.708 | 284 | 2.1x10⁻¹⁷ | -0.476 | 22.66% |
| PC ae C36:4 | PC ae C36:3 | 1.832 | 284 | 7.3x10⁻⁸ | -0.313 | 9.81% |
| PC ae C38:4 | PC ae C38:3 | 2.888 | 284 | 9.7x10⁻⁹ | -0.333 | 11.07% |
| PE aa C38:4 | PE aa C38:3 | 3.693 | 132 | 0.013 | -0.216 | 4.64% |
| PI aa C38:4* | PI aa C38:3* | 3.582 | 221 | 1.5x10⁻⁸ | -0.370 | 13.69% |

**Table 8: Metabolites associated (p<0.05) with genotype rs4775041 (LIPC) in the additive genetic model; in cases where alternative assignments of the metabolites are possible, these are indicated by a '*'. Full annotations can be found in the supplementary online data files. Reported are the mean concentrations (µM), standard deviation, the number of cases for which metabolite concentrations were obtained (ncases), the p-value of the association, the regression coefficient using an additive genetic model (estimate), and the measure of the observed variance that can be explained by the additive genetic model.**

| **Metabolite** | **mean** | **ncases** | **p-value** | **estimate** | **explained variance** |
|---|---|---|---|---|---|
| PE aa C38:6 | 3.921 | 284 | 9.7E-08 | 0.311 | 9.67% |
| PE aa C40:6 | 3.455 | 284 | 7.9E-06 | 0.263 | 6.89% |
| PE aa C38:5 | 3.024 | 284 | 4.2E-04 | 0.209 | 4.36% |
| PE aa C36:4 | 2.731 | 284 | 7.5E-04 | 0.200 | 3.98% |
| PC aa (COOH) C30:4 | 4.870 | 76 | 2.1E-03 | 0.347 | 12.04% |
| PE aa C38:4 | 5.357 | 284 | 3.2E-03 | 0.175 | 3.06% |
| PE aa C34:2 | 2.221 | 284 | 4.0E-03 | 0.171 | 2.93% |
| PC aa (COOH) C30:3* | 10.379 | 215 | 4.9E-03 | 0.192 | 3.68% |
| SM C16:0 | 77.224 | 284 | 1.4E-02 | 0.146 | 2.14% |
| PC ae C34:6* | 2.852 | 284 | 1.4E-02 | 0.145 | 2.12% |
| PE aa C40:5 | 1.575 | 132 | 1.5E-02 | 0.211 | 4.43% |
| TRP | 79.774 | 284 | 1.8E-02 | -0.140 | 1.97% |
| SM C16:1 | 9.982 | 284 | 1.8E-02 | 0.140 | 1.97% |
| SM C28:3 | 2.592 | 284 | 1.9E-02 | 0.139 | 1.94% |
| PC aa C38:6 | 146.588 | 284 | 2.1E-02 | 0.138 | 1.89% |
| SM (COOH) C16:2 | 2.926 | 284 | 2.3E-02 | 0.136 | 1.84% |
| SM C14:0 | 7.787 | 284 | 2.4E-02 | 0.135 | 1.81% |
| XLEU | 136.470 | 284 | 3.0E-02 | -0.129 | 1.67% |
| SM (OH,COOH) C14:1 | 2.162 | 221 | 3.1E-02 | 0.146 | 2.13% |
| PC ae C40:5 | 6.790 | 284 | 3.3E-02 | 0.127 | 1.62% |
| PI a (OH) C12:3 | 4.063 | 139 | 3.4E-02 | 0.181 | 3.28% |
| PE aa C36:2 | 4.422 | 284 | 3.4E-02 | 0.126 | 1.59% |
| VAL | 177.570 | 284 | 3.9E-02 | -0.123 | 1.51% |
| PC aa (COOH) C30:2 | 10.572 | 215 | 4.1E-02 | 0.140 | 1.97% |
| PC ae C38:6 | 11.669 | 284 | 4.1E-02 | 0.122 | 1.48% |
| PC ae (OH, COOH) | 10.582 | 215 | 4.1E-02 | 0.140 | 1.96% |
| C30:3 | | | | | |
| PC aa C40:6 | 46.067 | 284 | 4.4E-02 | 0.120 | 1.44% |
| PI aa C38:3* | 7.791 | 221 | 4.5E-02 | 0.136 | 1.84% |
| PE a (OH, COOH) C12:2 | 2.864 | 284 | 4.6E-02 | 0.119 | 1.41% |
| PE e (OH) C18:1 | 3.297 | 63 | 4.9E-02 | 0.249 | 6.22% |
| PC aa (COOH) C26:2* | 3.711 | 152 | 4.9E-02 | 0.161 | 2.60% |
| GLU | 129.766 | 284 | 4.9E-02 | -0.117 | 1.37% |

**Table 9: Metabolites associated (p<0.05) with genotype rs9309413 (PLEK) in the additive genetic model. In cases where alternative assignments of the metabolites are possible, these are indicated by a '*'. Full annotations can be found in the supplementary online data files. Reported are the mean concentrations (µM), standard deviation, the number of cases for which metabolite concentrations were obtained (ncases), the p-value of the association, the regression coefficient using an additive genetic model (estimate), and the measure of the observed variance that can be explained by the additive genetic model.**

| **metabolite** | **mean** | **ncases** | **p-value** | **estimate** | **explained variance** |
|---|---|---|---|---|---|
| SM C14:0 | 7.79 | 284 | 1.95E-09 | 0.347 | 12.01% |
| SM C16:0 | 77.22 | 284 | 4.34E-04 | 0.207 | 4.30% |
| PC ae C42:3 | 1.56 | 132 | 1.31 E-03 | 0.277 | 7.67% |
| SM C22:1 | 13.76 | 284 | 1.95E-03 | 0.183 | 3.35% |
| SM (COOH) C12:0 | 0.96 | 208 | 1.97E-03 | 0.213 | 4.55% |
| SM C18:0* | 16.46 | 284 | 3.35E-03 | 0.174 | 3.01% |
| SM C26:4 | 3.51 | 284 | 3.94E-03 | 0.171 | 2.91% |
| PC aa (COOH) C26:2* | 3.71 | 152 | 5.69E-03 | 0.223 | 4.99% |
| SM C24:4 | 118.12 | 60 | 6.42E-03 | -0.348 | 12.12% |
| SM C24:3 | 3.89 | 284 | 7.47E-03 | 0.158 | 2.51% |
| PC aa C28:1* | 4.72 | 69 | 7.48E-03 | 0.319 | 10.20% |
| PC aa (OH, COOH) | 6.61 | 284 | 7.59E-03 | 0.158 | 2.50% |
| C28:4 | | | | | |
| PI aa (OH, COOH) | 24.13 | 139 | 7.80E-03 | -0.225 | 5.05% |
| C28:2 | | | | | |
| SM (COOH) C18:1 | 7.84 | 284 | 1.02E-02 | 0.152 | 2.32% |
| SM C18:1 | 7.47 | 284 | 1.07E-02 | 0.151 | 2.29% |
| SM C24:2 | 16.82 | 221 | 1.11E-02 | 0.171 | 2.91% |
| PC ae C38:1* | 11.49 | 284 | 1.21 E-02 | 0.149 | 2.21% |
| PE aa (COOH) C14:1 | 1.40 | 63 | 1.26E-02 | 0.313 | 9.78% |
| PE ae (OH, COOH) | 1.43 | 63 | 1.27E-02 | 0.312 | 9.76% |
| C14:2 | | | | | |
| PC ae C40:5 | 6.79 | 284 | 1.33E-02 | 0.147 | 2.15% |
| SM C22:3 | 2.99 | 208 | 1.34E-02 | 0.171 | 2.93% |
| SM C20:4 | 206.98 | 60 | 1.52E-02 | -0.312 | 9.74% |
| PC ae C40:4 | 4.81 | 284 | 1.83E-02 | 0.140 | 1.96% |
| PC ae C38:0* | 5.24 | 284 | 1.98E-02 | 0.138 | 1.91% |
| SM C20:3 | 10.73 | 284 | 2.18E-02 | 0.136 | 1.85% |
| SM (OH) C20:1 | 16.74 | 284 | 2.58E-02 | 0.132 | 1.75% |
| PC ae C36:2 | 25.33 | 284 | 2.59E-02 | 0.132 | 1.75% |
| PC ae C32:7* | 10.96 | 221 | 2.60E-02 | 0.150 | 2.24% |
| SM C20:1 | 4.18 | 284 | 2.61 E-02 | 0.132 | 1.74% |
| PE e C14:0 | 82.97 | 132 | 2.75E-02 | 0.192 | 3.68% |
| PC aa (COOH) C30:3 | 10.38 | 215 | 2.79E-02 | 0.150 | 2.25% |
| PC ae (OH, COOH) | 10.38 | 215 | 2.79E-02 | 0.150 | 2.25% |
| C30:4 | | | | | |
| Docosahexaonic acid | 4.14 | 283 | 3.31 E-02 | 0.127 | 1.61% |
| PC aa C36:2 | 412.59 | 284 | 3.41 E-02 | 0.126 | 1.58% |
| SM C26:3 | 3.28 | 284 | 3.55E-02 | 0.125 | 1.56% |
| PC aa (OH, COOH) | 342.95 | 284 | 3.70E-02 | 0.124 | 1.53% |
| C30:4 | | | | | |
| SM (COOH) C16:0 | 3.31 | 284 | 4.06E-02 | 0.122 | 1.48% |
| PC ae C32:0* | 7.89 | 284 | 4.67E-02 | 0.118 | 1.40% |
| PC aa (COOH) C14:2 | 5.22 | 215 | 4.70E-02 | 0.136 | 1.84% |
| SM C22:0 | 21.51 | 221 | 4.77E-02 | 0.133 | 1.78% |
| PC ae (COOH) C30:3 | 7.37 | 208 | 4.80E-02 | 0.137 | 1.88% |
| PC a C18:1* | 15.55 | 284 | 4.92E-02 | 0.117 | 1.36% |

**Table 10: Metabolites associated (p<0.05) with genotype rs1148259 (ANKRD30A) in the additive genetic model. In cases where alternative assignments of the metabolites are possible, these are indicated by a '*'. Full annotations can be found in the supplementary online data files. Reported are the mean concentrations (µM), standard deviation, the number of cases for which metabolite concentrations were obtained (ncases), the p-value of the association, the regression coefficient using an additive genetic model (estimate), and the measure of the observed variance that can be explained by the additive genetic model.**

| **metabolite** | **mean** | **ncases** | **p-value** | **estimate** | **explained variance** |
|---|---|---|---|---|---|
| SM (OH,COOH) C18:2 | 92.45 | 284 | 3.04E-09 | 0.343 | 11.74% |
| SM (OH,COOH) C16:1 | 27.23 | 284 | 6.40E-07 | 0.290 | 8.43% |
| SM (OH,COOH) C18:1 | 19.27 | 284 | 7.81 E-07 | 0.288 | 8.30% |
| SM (OH,COOH) C16:2* | 84.94 | 284 | 1.46E-06 | 0.281 | 7.91% |
| SM (OH,COOH) C22:0 | 17.32 | 284 | 2.67E-06 | 0.274 | 7.53% |
| SM (OH) C22:1 | 44.57 | 284 | 5.59E-06 | 0.266 | 7.06% |
| SM (OH,COOH) C20:2 | 64.74 | 284 | 7.61 E-06 | 0.262 | 6.87% |
| SM (OH) C20:3 | 172.87 | 284 | 2.30E-05 | 0.248 | 6.17% |
| SM (OH,COOH) C20:4 | 74.88 | 284 | 3.13E-05 | 0.244 | 5.97% |
| SM (OH,COOH) C24:0 | 20.93 | 284 | 5.78E-05 | 0.236 | 5.58% |
| SM (OH,COOH) C16:0 | 56.42 | 284 | 6.17E-05 | 0.235 | 5.54% |
| SM (OH,COOH) C20:3 | 45.92 | 284 | 7.10E-05 | 0.234 | 5.45% |
| SM (OH) C22:0 | 17.06 | 284 | 7.73E-05 | 0.232 | 5.40% |
| SM (OH) C22:2 | 31.89 | 284 | 8.05E-05 | 0.232 | 5.37% |
| SM (OH) C24:0 | 11.80 | 208 | 1.14E-04 | 0.264 | 6.99% |
| SM (OH,COOH) C24:1 | 22.98 | 284 | 1.88E-04 | 0.220 | 4.83% |
| SM (OH) C20:1 | 16.74 | 284 | 3.04E-04 | 0.213 | 4.53% |
| SM (OH) C26:0 | 30.79 | 284 | 7.70E-04 | 0.198 | 3.94% |
| SM (OH) C20:0 | 25.82 | 284 | 1.23E-03 | 0.191 | 3.64% |
| PE a C10:0 | 4.16 | 284 | 1.26E-03 | 0.191 | 3.63% |
| PE e (COOH) C12:1* | 42.07 | 284 | 1.32E-03 | 0.190 | 3.60% |
| SM (COOH) 18:0 | 21.85 | 221 | 1.55E-03 | 0.212 | 4.48% |
| SM (OH) C28:0 | 59.33 | 284 | 2.44E-03 | 0.179 | 3.21% |
| SM (OH,COOH) C20:0 | 13.60 | 132 | 2.79E-03 | 0.258 | 6.67% |
| PC aa C34:4 | 3.25 | 284 | 3.06E-03 | 0.175 | 3.07% |
| SM (OH,COOH) C20:1 | 10.98 | 69 | 4.61 E-03 | 0.337 | 11.37% |
| SM (OH) C28:1 | 24.76 | 284 | 6.80E-03 | 0.160 | 2.57% |
| PG aa (OH, COOH) C20:2 | 17.35 | 63 | 7.45E-03 | 0.334 | 11.16% |
| PE e C16:3* | 4.72 | 139 | 1.59E-02 | 0.204 | 4.17% |
| PC aa C36:5 | 47.53 | 284 | 1.93E-02 | 0.139 | 1.93% |
| PC ae C36:0 | 3.42 | 284 | 2.13E-02 | 0.137 | 1.87% |
| PG a (OH, COOH) C14:3 | 290.41 | 76 | 2.24E-02 | 0.262 | 6.85% |
| PE aa (OH, COOH) C24:2 | 4.58 | 63 | 2.52E-02 | -0.282 | 7.94% |
| SM (OH,COOH) C18:0 | 35.99 | 63 | 3.07E-02 | 0.273 | 7.43% |
| PE e C16:3 | 5.27 | 63 | 3.10E-02 | 0.272 | 7.40% |
| PC aa C38:4 | 209.05 | 284 | 3.24E-02 | 0.127 | 1.61% |
| PC a C20:4 | 5.09 | 284 | 3.32E-02 | 0.126 | 1.60% |
| GalCer sulf C18:0 | 31.85 | 61 | 3.81 E-02 | 0.266 | 7.09% |
| PC aa C36:4 | 399.41 | 284 | 3.92E-02 | 0.122 | 1.50% |
| PC aa C30:2 | 8.99 | 284 | 3.96E-02 | 0.122 | 1.49% |
| SM (COOH) 18:1 | 15.29 | 152 | 4.02E-02 | 0.167 | 2.78% |
| PIP aa C30:2 | 2.92 | 76 | 4.21 E-02 | 0.234 | 5.46% |
| PC a C26:1 | 0.91 | 63 | 4.93E-02 | 0.249 | 6.19% |
| PC aa C38:3 | 123.00 | 284 | 4.97E-02 | 0.117 | 1.36% |

**Table 11: Metabolites associated (p<0.05) with genotype rs992037 (PARK2) in the additive genetic model. In cases where alternative assignments of the metabolites are possible, these are indicated by a '*'. Full annotations can be found in the supplementary online data files. Reported are the mean concentrations (µM), standard deviation, the number of cases for which metabolite concentrations were obtained (ncases), the p-value of the association, the regression coefficient using an additive genetic model (estimate), and the measure of the observed variance that can be explained by the additive genetic model.**

| **metabolite** | **mean** | **ncases** | **p-value** | **estimate** | **explained variance** |
|---|---|---|---|---|---|
| LYS | 215.17 | 284 | 1.20E-07 | 0.308 | 9.48% |
| PC aa C38:1* | 18.72 | 284 | 3.39E-03 | 0.173 | 3.00% |
| GLY | 162.50 | 284 | 5.28E-03 | 0.165 | 2.73% |
| HIS | 76.52 | 284 | 6.17E-03 | 0.162 | 2.63% |
| C16:1 | 0.04 | 284 | 6.19E-03 | -0.162 | 2.63% |
| GLU | 129.77 | 284 | 7.02E-03 | -0.160 | 2.55% |
| C6 | 0.09 | 284 | 8.41 E-03 | -0.156 | 2.44% |
| PC aa C42:6 | 1.27 | 63 | 9.04E-03 | -0.326 | 10.65% |
| SM (OH,COOH) C20:2 | 64.74 | 284 | 9.06E-03 | 0.155 | 2.39% |
| C14:1 | 0.13 | 284 | 1.03E-02 | -0.152 | 2.31% |
| C18:2-OH | 0.05 | 284 | 1.18E-02 | -0.149 | 2.23% |
| Docosahexaonic acid | 4.14 | 283 | 1.32E-02 | -0.147 | 2.16% |
| SM (OH,COOH) C24:0 | 20.93 | 284 | 1.49E-02 | 0.144 | 2.09% |
| PC ae C22:8 | 11.53 | 76 | 1.72E-02 | 0.273 | 7.43% |
| ARG | 115.97 | 284 | 2.01 E-02 | 0.138 | 1.90% |
| ORN | 57.03 | 284 | 2.27E-02 | 0.135 | 1.83% |
| PI a (OH, COOH) C14:2 | 3.74 | 76 | 2.37E-02 | -0.259 | 6.73% |
| Methionine Sulfonate | 2.39 | 284 | 2.45E-02 | 0.133 | 1.78% |
| C2 | 8.60 | 284 | 2.59E-02 | -0.132 | 1.75% |
| PA aa C42:5 | 84.31 | 284 | 2.62E-02 | -0.132 | 1.74% |
| C18:1 | 0.16 | 284 | 2.99E-02 | -0.129 | 1.66% |
| C12:1 | 0.12 | 284 | 3.06E-02 | -0.128 | 1.65% |
| PE aa C40:5 | 1.57 | 132 | 3.76E-02 | -0.181 | 3.28% |
| SM (OH,COOH) C18:1 | 19.27 | 284 | 3.76E-02 | 0.123 | 1.52% |
| SM (OH,COOH) C14:1 | 2.16 | 221 | 3.91 E-02 | -0.139 | 1.93% |
| PC a C22:5 | 0.81 | 63 | 4.13E-02 | -0.258 | 6.65% |
| PC ae C38:0* | 5.24 | 284 | 4.35E-02 | -0.120 | 1.44% |
| C10 | 0.32 | 284 | 4.35E-02 | -0.120 | 1.44% |
| SM C28:3 | 2.59 | 284 | 4.87E-02 | 0.117 | 1.37% |
| SM (OH,COOH) C18:2 | 92.45 | 284 | 4.95E-02 | 0.117 | 1.36% |

**Table 12: Selected metabolite concentration ratios associated (p<0.05) with genotype rs992037 (PARK2) in the additive genetic model (see Table S2 for legend; ncases=284). The improvement of the p-value of association when using metabolite concentration ratios is calculated with respect to the larger of the two p-values for the individual metabolites. *The "improvement of p-value" is computed using the following formula: min(p[C_enumerator], p[C_nominator]) / p[C_enumerator / C_nominator], where C_ is a metabolite concentration and p[.]the corresponding p-value of association.**

| **enumerator** | **nominator** | **Mean** | **p-value** | **estimate** | **improvement of p-value*** |
|---|---|---|---|---|---|
| ARG | GLU | 0.927 | 4.73E-06 | 0.268 | 1484.4 |
| MET | GLU | 0.333 | 5.75E-06 | 0.265 | 1221.3 |
| LYS | GLU | 1.773 | 6.79E-06 | 0.263 | 0.018 |
| GLY | GLU | 1.305 | 1.07E-05 | 0.258 | 491.1 |
| ORN | GLU | 0.455 | 1.16E-05 | 0.257 | 604.8 |
| HIS | GLU | 0.613 | 2.37E-05 | 0.248 | 260.0 |
| TRP | GLU | 0.629 | 3.20E-05 | 0.244 | 219.2 |
| THR | GLU | 0.540 | 5.86E-05 | 0.236 | 119.8 |
| PHE | GLU | 0.575 | 5.94E-05 | 0.236 | 118.1 |
| MET.SULF | GLU | 0.019 | 1.21 E-04 | 0.226 | 58.1 |
| TYR | GLU | 0.514 | 2.60E-04 | 0.215 | 27.0 |
| ALA | GLU | 2.248 | 4.45E-04 | 0.207 | 15.8 |
| CIT | GLU | 0.152 | 1.28E-03 | 0.190 | 5.5 |

### References

Altmaier E, Ramsay SL, Graber A, Mewes HW, Weinberger KM et al. (2008) Bioinformatics analysis of targeted metabolomics - uncovering old and new tales of diabetic mice under medication. Endocrinology 149: 34783489.
Assfalg M, Bertini I, Colangiuli D, Luchinat C, Schafer H et al. (2008) Evidence of different metabolic phenotypes in humans. Proc Natl Acad Sci U S A 105(5): 1420-1424.
Brookes KJ, Chen W, Xu X, Taylor E, Asherson P (2006) Association of fatty acid desaturase genes with attention-deficit/hyperactivity disorder. Biol Psychiatry 60(10): 1053-1061.
Caspi A, Williams B, Kim-Cohen J, Craig IW, Milne BJ et al. (2007) Moderation of breastfeeding effects on the IQ by genetic variation in fatty acid metabolism. Proc Natl Acad Sci U S A 104(47): 18860-18865.
D6ring A, Gieger C, Mehta D, Gohlke H, Prokisch H et al. (2008) SLC2A9 influences uric acid concentrations with pronounced sex-specific effects. Nat Genet 40(4): 430-436.
Heid IM, Boes E, Müller AM, Kollerits B, Lamina C et al. (2008) Genome-wide association analysis of high-density lipoprotein cholesterol in the population-based KORA Study sheds new light on intergenic regions. Circ Cardiovasc Genetics 1:10-20.
Kathiresan S, Melander O, Guiducci C, Surti A, Burtt NP et al. (2008) Six new loci associated with blood low-density lipoprotein cholesterol, high-density lipoprotein cholesterol or triglycerides in humans. Nat Genet 40(2): 189-197.
Kanehisa M, Goto S, Hattori M, Aoki-Kinoshita KF, Itoh M et al. (2006) From genomics to chemical genomics: new developments in KEGG. Nucleic Acids Res 34 (Database issue): D354-357.
Lindon JC, Holmes E, Nicholson JK (2007) Metabonomics in pharmaceutical R&D. Febs J 274(5): 1140-1151.
Maier EM, Liebl B, Roschinger W, Nennstiel-Ratzel U, Fingerhut R et al. (2005) Population spectrum of ACADM genotypes correlated to biochemical phenotypes in newborn screening for medium-chain acyl-CoA dehydrogenase deficiency. Hum Mutat 25(5): 443-452.
Malerba G, Schaeffer L, Xumerle L, Klopp N, Trabetti E et al. (2008) SNPs of the FADS Gene Cluster are Associated with Polyunsaturated Fatty Acids in a Cohort of Patients with Cardiovascular Disease. Lipids 43(4): 289-299.
Nagan N., Kruckeberg KE, Tauscher AL, Bailey KS, Rinaldo P, Matern D. (2003) The frequency of short-chain acyl-CoA dehydrogenase gene variants in the US population and correlation with the C(4)-acylcarnitine concentration in newborn blood spots. Mol Genet Metab. 78(4):239-46.
Samani NJ, Erdmann J, Hall AS, Hengstenberg C, Mangino M et al. (2007) Genomewide association analysis of coronary artery disease. N Engl J Med 357(5): 443-453.
Schaeffer L, Gohlke H, Muller M, Heid IM, Palmer LJ et al. (2006) Common genetic variants of the FADS1 FADS2 gene cluster and their reconstructed haplotypes are associated with the fatty acid composition in phospholipids. Hum Mol Genet 15(11): 1745-1756.
Wallace C, Newhouse SJ, Braund P, Zhang F, Tobin M et al. (2008) Genome-wide association study identifies genes for biomarkers of cardiovascular disease: serum urate and dyslipidemia. Am J Hum Genet 82: 139-149.
Wichmann HE, Gieger C, Illig T (2005) KORA-gen--resource for population genetics, controls and a broad spectrum of disease phenotypes. Gesundheitswesen 67 Suppl 1: S26-30.
Willer CJ, Sanna S, Jackson AU, Scuteri A, Bonnycastle LL et al. (2008) Newly identified loci that influence lipid concentrations and risk of coronary artery disease. Nat Genet 40(2): 161-169.
Wishart DS, Tzur D, Knox C, Eisner R, Guo AC et al. (2007) HMDB: the Human Metabolome Database. Nucleic Acids Res 35(Database issue): D521-526.
WTCCC (2007) Genome-wide association study of 14,000 cases of seven common diseases and 3,000 shared controls. Nature 447(7145): 661-678.
Zeggini E, Scott LJ, Saxena R, Voight BF, Marchini JL et al. (2008) Meta-analysis of genome-wide association data and large-scale replication identifies additional susceptibility loci for type 2 diabetes. Nat Genet 40(5): 638-645.

## Claims

1. A method for determining a predisposition of a human subject for physiological susceptibilities that result from alterations in lipid metabolism, comprising determining, in a sample obtained from the human subject, the genotype of that person with respect to at least two genetic polymorphisms selected from the group consisting of
a) rs2014355; wherein the minor allele is represented by a cytidine and the major allele is represented by a thymidine;
b) rs11161510; wherein the minor allele is represented by a thymidine and the major allele is represented by a cytidine;
c) rs2286963; wherein the minor allele is represented by a guanosine and the major allele is represented by a thymidine;
d) rs174548; wherein the minor allele is represented by a guanosine and the major allele is represented by a cytidine;
e) rs9393903; wherein the minor allele is represented by a adenosine and the major allele is represented by a guanosine;
f) rs168622; wherein the minor allele is represented by a thymidine and the major allele is represented by a guanosine;
g) rs541503; wherein the minor allele is represented by a cytidine and the major allele is represented by a thymidine;
h) rs2046813; wherein the minor allele is represented by a cytidine and the major allele is represented by a thymidine;
i) rs272889; wherein the minor allele is represented by a adenosine and the major allele is represented by a guanosine; and
j) at least one genetic polymorphism that is in linkage disequilibrium with any of the genetic polymorphisms of (a) to (i),
wherein the presence of one or two copies of the minor allele of at least two genetic polymorphisms is indicative of a predisposition for said physiological susceptibilities.

2. The method of claim 1, wherein
a) the presence of one or two copies of the minor allele of rs2014355 or a genetic polymorphism in linkage disequilibrium thereto is associated with a decreased yield of the enzymatic reactions that are catalyzed by SCAD (EC:1.3.99.2);
b) the presence of one or two copies of the minor allele of rs11161510 or a genetic polymorphism in linkage disequilibrium thereto is associated with a decreased yield of the enzymatic reactions that are catalyzed by MCAD (EC:1.3.99.3);
c) the presence of one or two copies of the minor allele of rs2286963 or a genetic polymorphism in linkage disequilibrium thereto is associated with a increased yield of the enzymatic reactions that are catalyzed by LCAD (EC:1.3.99.13 and EC:1.3.99.3);
d) the presence of one or two copies of the minor allele of rs174548 or a genetic polymorphism in linkage disequilibrium thereto is associated with a decreased yield of the enzymatic reactions that are catalyzed by FADS1 (EC:1.14.99.25);
e) the presence of one or two copies of the minor allele of rs9393903 or a genetic polymorphism in linkage disequilibrium thereto is associated with a decreased yield of the enzymatic reactions that are catalyzed by ELOVL2 (EC:2.3.1.-);
f) the presence of one or two copies of the minor allele of rs168622 or a genetic polymorphism in linkage disequilibrium thereto is associated with an increased affinity for longer chain sphingomyelins of the regulatory subunit SPTLC3 in the SPT enzymatic complex (EC:2.3.1.50);
g) the presence of one or two copies of the minor allele of rs541503 or a genetic polymorphism in linkage disequilibrium thereto is associated with a increased yield of the enzymatic reaction that is catalyzed by PHGDH (EC:1.1.1.95);
h) the presence of one or two copies of the minor allele of rs2046813 or a genetic polymorphism in linkage disequilibrium thereto is associated with an increased substrate affinity for longer chain fatty acids of the enzymatic reactions that are catalyzed by ACSL1 (EC:6.2.1.3);
i) the presence of one or two copies of the minor allele of rs272889 or a genetic polymorphism in linkage disequilibrium thereto is associated with an increased transporter activity of C5-Acylcarnitine that is catalyzed by OCTN1.

3. The method of claim 1 or 2, wherein the genotype of at least three genetic polymorphisms is determined.

4. The method of any one of claims 1 to 3, wherein the genotype of at least the genetic polymorphisms of (a) to (i) is determined.

5. The method of any one of claims 1 to 4, wherein the genetic polymorphisms are selected from the group consisting of single nucleotide polymorphisms, insertions or deletions.

6. The method of any one of claims 1 to 5, wherein the genotype of the genetic polymorphisms is detected by PCR based techniques, DNA sequencing-based techniques, hybridization-based techniques, single-strand conformation polymorphism analysis (SSCA), denaturating gradient gel electrophoresis (DGGE), mismatch cleavage detection, heteroduplex analysis, primer extension-based techniques or 5'-nuclease assay-based techniques.

7. The method of any one of claims 1 to 6, wherein the genotype of the genetic polymorphisms is detected using a solid phase support.

8. The method according to any one of claims 1 to 7, wherein the physiological susceptibilities are selected from sensitivity to drug treatment, functional food, physical health schemes, identification of non-responsiveness to treatment by diet, medication or physical activity.

9. The method according to any one of claims 1 to 8, wherein the physiological susceptibilities results in a disease or condition selected from hyperactivity, a potential benefit from a specific nutrition (e.g. breast feeding and IQ) but also type 2 diabetes, metabolic syndrome, coronary artery disease, Crohn's disease, rheumatic arthritis, border line syndrome and increased levels of cholesterol and triglycerides.

10. The method of any one of claims 1 to 9, wherein the sample is selected from blood, serum, plasma, fetal tissue, saliva, urine, mucosal tissue, mucus, vaginal tissue, fetal tissue obtained from the vagina, skin, hair or hair follicle.

11. A kit for determining a predisposition of a subject for physiological susceptibilities that result from alterations in lipid metabolism comprising a set of probes, wherein the set comprises at least two probes selected from the group consisting of:
a) a probe specifically binding to rs2014355;
b) a probe specifically binding to rs11161510;
c) a probe specifically binding to rs2286963;
d) a probe specifically binding to rs174548;
e) a probe specifically binding to rs9393903;
f) a probe specifically binding to rs168622;
g) a probe specifically binding to rs541503;
h) a probe specifically binding to rs2046813;
i) a probe specifically binding to rs272889; and
j) at least one probe specifically binding to a genetic polymorphism that is in linkage disequilibrium with a genetic polymorphism selected from the group consisting of rs2014355; rs11161510; rs2286963; rs174548; rs9393903; rs168622; rs541503; rs2046813 and rs272889.

12. The kit according to claim 11, wherein the set of probes is provided on a solid phase support.
